(19) Europäisches Patentamt
European Patent Office
Office européen des brevets

(11) EP 1 525 010 B1

(12) EUROPEAN PATENT SPECIFICATION

(45) Date of publication and mention of the grant of the patent:
**01.10.2014 Bulletin 2014/40**

(21) Application number: **03771650.3**

(22) Date of filing: **18.07.2003**

(51) Int Cl.:
***A61F 13/532*** (2006.01) ***A61F 13/536*** (2006.01)
***A61F 13/537*** (2006.01) ***A61F 13/539*** (2006.01)
***A61F 13/84*** (2006.01) ***A61F 13/00*** (2006.01)
***A61F 13/15*** (2006.01) ***B01D 53/26*** (2006.01)
***B01D 53/28*** (2006.01) ***C09D 133/14*** (2006.01)
***C08L 71/02*** (2006.01) ***A61L 15/60*** (2006.01)

(86) International application number:
**PCT/US2003/022424**

(87) International publication number:
**WO 2004/011046 (05.02.2004 Gazette 2004/06)**

(54) **ABSORBENT BINDER COMPOSITION, METHOD OF MAKING IT, AND ARTICLES INCORPORATING IT**

ABSORBIERENDE BINDEMITTEL-ZUSAMMENSETZUNG, VERFAHREN ZU DEREN HERSTELLUNG UND ARTIKEL DIE DIESE ENTHALTEN

COMPOSITION DE LIANT ABSORBANT, PROCEDE DE FABRICATION DE CETTE COMPOSITION ET ARTICLES DANS LESQUELS EST INCORPOREE CETTE COMPOSITION

(84) Designated Contracting States:
**DE GB NL SE**

(30) Priority:
**26.07.2002 US 206883**
**20.12.2002 US 324478**
**01.05.2003 US 427809**
**01.05.2003 US 427700**
**01.05.2003 US 427564**
**01.05.2003 US 427808**

(43) Date of publication of application:
**27.04.2005 Bulletin 2005/17**

(73) Proprietor: **KIMBERLY-CLARK WORLDWIDE, INC.**
**Neenah, WI 54956 (US)**

(72) Inventors:
- **SOERENS, Dave, Allen**
  **Neenah, WI 54956 (US)**
- **LAUMER, Jason, Matthew**
  **Appleton, WI 54915 (US)**
- **MAKOUI, Kambiz, Bayat**
  **Neenah, WI 54956 (US)**
- **DRAVES, Julie, Anne**
  **Appleton, Wisconsin 54913 (US)**
- **SAWYER, Lawrence, Howell**
  **Neenah, WI 54956 (US)**
- **MELIUS, Shannon, Kathleen**
  **Appleton, WI 54914 (US)**
- **MCDOWALL, Debra, Jean**
  **Neenah, WI 54956 (US)**
- **EVERETT, Rob, D.**
  **Appleton, WI 54911 (US)**
- **GRIESBACH, Henry, L. III**
  **Clarkston, GA 30021 (US)**
- **NELSON, Brenda, Marie**
  **Appleton, WI 54915 (US)**
- **KRAUTKRAMER, Candace, Dyan**
  **Neenah, WI 54956 (US)**
- **GEORGE, Russell, Paul**
  **Appleton, WI 54914 (US)**
- **CASSON, Kenneth, Russell**
  **Menasha, Wisconsin 54952 (US)**
- **WANG, James, Hongxue**
  **Appleton, WI 54911 (US)**
- **UTTECHT, Cathleen, Mae**
  **Menasha, WI 54952 (US)**

(74) Representative: **Beacham, Annabel Rose**
**Dehns**
**St Bride's House**
**10 Salisbury Square**
**London EC4Y 8JD (GB)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

(Cont. next page)

(56) References cited:
**EP-A- 0 132 910** **EP-A- 0 705 861**
**EP-A- 0 844 265** **EP-A- 1 059 320**
**WO-A-02/053664** **US-B1- 6 403 857**

## Description

BACKGROUND OF THE INVENTION

This invention is directed to an absorbent binder or coating composition, a method of making the absorbent binder or coating composition, and absorbent articles which utilize the composition.

Adhesives, or binders, are a necessary element of many absorbent products. While adhesives beneficially hold products together, adhesives may also have a tendency to interfere with the absorbency of fluids in absorbent products. Adhesives are typically hydrophobic and therefore are not conducive to absorbency or liquid transfer functions. Furthermore, most adhesives are non-absorbent and thus serve no liquid retention function

Hydrophilic adhesives are known, such as adhesives formulated from water-soluble polymers such as poly(vinyl alcohol), poly(vinyl methyl ether), poly(vinyl pyrrolidone), poly(ethylene oxide), or cellulose derivatives such as hydroxypropyl cellulose. Dextrans, starches and vegetable gums have been used to provide hydrophilic adhesives. These materials provide adhesion under dry conditions. However, upon exposure to aqueous fluids, these materials lose bonding capability because they are substantially soluble in aqueous fluids.

A known approach for making hydrophilic adhesives more functional upon exposure to aqueous fluid is to crosslink the water-soluble polymers. As a result of crosslinking, the material becomes swellable, and no longer soluble, in aqueous fluid. However, crosslinked polymers are difficult to apply to substrates or to establish intimate contact with surfaces because the crosslinked polymers are solid materials and have little or no ability to flow.

What is therefore needed is a hydrophilic binder or coating that has latent crosslinking capability. Such binder or coating could be easily applied, like a water-soluble polymer, since the hydrophilic binder or coating would be capable of flow prior to crosslinking. Latent crosslinking capability would also provide a simple means of crosslinking the polymer after the polymer has established intimate contact with substrates or has formed a desired final shape or form.

Post-application crosslinking is well known. Typical means of inducing the formation of crosslinks include high temperature "curing" or exposure to radiation, such as ultraviolet or gamma radiation. Another known means of post-application crosslinking is moisture-induced crosslinking.

Recent development efforts have provided coating materials for a variety of uses. For example, U.S. Patent No. 6,054,523, to Braun et al., describes materials that are formed from organopolysiloxanes containing groups that are capable of condensation, a condensation catalyst, an organopolysiloxane resin, a compound containing a basic nitrogen, and polyvinyl alcohol. The materials are reported to be suitable for use as hydrophobic coatings and for paints and sealing compositions.

Anderson et al., in U.S. Patent No. 5,196,470, reported an alcohol-based, water-soluble binder composition. Because this composition is water-soluble and not cross-linked, it has no absorbency.

Others have reported the production of graft copolymers having silane functional groups that permitted the initiation of cross-linking by exposure to moisture. Prejean (U. S. Patent No. 5,389,728) describes a melt-processible, moisture-curable graft copolymer that was the reaction product of ethylene, a 1 - 8 carbon alkyl acrylate or methacrylate, a glycidyl containing monomer such as glycidyl acrylate or methacrylate, onto which has been grafted N-tert-butylaminopropyl trimethoxysilane. The resulting copolymers were reported to be useful as adhesives and for wire and cable coatings.

Furrer et al., in U.S. Patent No. 5,112,919, reported a moisture-crosslinkable polymer that was produced by blending a thermoplastic base polymer, such as polyethylene, or a copolymer of ethylene, with 1-butene, 1-hexene, 1-octene, or the like; a solid carrier polymer, such as ethylene vinylacetate copolymer (EVA), containing a silane, such as vinyltrimethoxysilane; and a free-radical generator, such as an organic peroxide; and heating the mixture. The copolymers could then be cross-linked by reaction in the presence of water and a catalyst, such as dibutyltin dilaurate, or stannous octoate.

U.S. Patent No. 4,593,071 to Keough reported moisture cross-linkable ethylene copolymers having pendant silane acryloxy groups. The resultant cross-linked polymers were reported to be especially resistant to moisture and to be useful for extruded coatings around wires and cables. The same group has reported similar moisture curable polymers involving silanes in U.S. Patent Nos. 5,047,476, 4,767,820, 4,753,993, 4,579,913, 4,575,535, 4,551,504, 4,526,930, 4,493,924, 4,489,029, 4,446,279, 4,440,907, 4,434,272, 4,408,011, 4,369,289, 4,353,997, 4,343,917, 4,328,323, and 4,291,136.

WO 02/053644 discloses absorbent coatings comprising a water soluble base polymer having graft polymerized thereto an organic moiety that includes a group which reacts with water to form a silanol group.

U.S. Patent No. 5,204,404 to Werner reported crosslinkable hydrophobic acrylate ester copolymers including 0.1 to 10% acrylic acid. The resultant cross-linked polymers were reported to be useful for painting and refinishing the exterior of automobiles.

These examples of moisture-induced crosslinking are applied to substantially hydrophobic polymers. Since the cured products of these formulations are reported to be useful for coverings for wire and cable, and for non-conductive

coatings for electrical conductors, and for painting and refinishing the exterior of automobiles, it would be expected that they are durable coatings for which properties such as water absorbency would be a disadvantage.

There is thus a need within the field of absorbent products for absorbent binders, adhesives, or coatings. Furthermore, there is a need within the field of absorbent products for such absorbent binders, adhesives, or coatings that can be prepared by post-application, moisture-induced crosslinking of hydrophilic polymers.

## SUMMARY OF THE INVENTION

In response to the discussed difficulties and problems encountered in the prior art, a new absorbent composition as defined in claim 1, useful as a binder, adhesive, or coating material, has been discovered. The absorbent composition includes a hydrophilic polymer which has the capability of post-application, moisture-induced crosslinking. This capability provides for absorbent products having greater absorbent capacity.

The absorbent binder composition according to claim 1 comprises a water-soluble ionic polymer capable of sufficient spontaneous crosslinking within 10 minutes, at a temperature of 120°C or less, to reach an absorbent capacity of at least one gram of fluid per gram of absorbent binder composition. Thereafter, the ionic polymer continues to crosslink until full absorbent capacity is reached. The ionic polymer may bear a positive or negative charge, or both.

The absorbent binder composition is defined in claim 1 and includes at least 15 mass percent monoethylenically unsaturated monomer or polymer containing the monomer, selected 30 from carboxylic acid, sulphonic acid, phosphonic acid, or salts thereof, or a quaternary ammonium salt; and a second monomer. The second monomer is an acrylate or methacrylate ester containing an alkoxysilane functionality, which group is readily transformed into a silanol functionality by subsequent reaction with water. Upon exposure to water, the silanol functional group condenses to form a crosslinked polymer. Thus, the absorbent binder composition provides enhanced adhesion in a wet condition, as well as absorbency.

The flexible absorbent binder composition (useful in a wide variety of applications requiring flexibility and conformability) includes 15 to 99.8% by mass of monoethylenically unsaturated polymer units formed by monomers selected from carboxylic acid, sulphonic acid, phosphonic acid, salts of the foregoing, and quaternary ammonium salts. The flexible absorbent binder composition also includes 0.1 to 20% by mass of acrylate or methacrylate ester units that include an alkoxysilane functionality. Upon exposure to water, the alkoxysilane functionality forms a silanol group which condenses to form a crosslinked polymer.

The flexible absorbent binder composition also includes 0.1 to 75% by mass of polyolefin glycol and/or polyolefin oxide units. The polyolefin glycol and/or oxide may include an alpha-olefin having 2 to 4 carbon atoms, and may include 30 to 15,000 olefin glycol and/or oxide units per molecule. The polyolefin glycol and/or oxide may be graft polymerized with the acrylate or methacrylate ester to form a graft copolymer. The polyolefin glycol and/or oxide may be a homopolymer or copolymer. The polyolefin glycol and/or oxide may be a block copolymer including olefin glycol or oxide units having different numbers of carbon atoms, for instance, block copolymers of ethylene oxide and propylene oxide. The polyolefin glycol and/or oxide provides the absorbent binder composition with enhanced flexibility. Thus, the absorbent binder composition has enhanced adhesion in a wet condition, absorbency, and flexibility.

The absorbent binder composition suitably has a glass transition temperature below about 30 degrees Celsius, or below about 10 degrees Celsius, and a bending modulus lower than the bending modulus of a substrate to which the composition is applied. The absorbent binder composition may be used in the manufacture of absorbent products, and therefore may be applied to such substrates as nonwoven webs, woven webs, knitted fabrics, cellulose tissue, plastic film, stranded composites, elastomer net composites, or any other suitable substrates. Examples of suitable types of plastic film substrates include those made of polypropylene, low density polyethylene, high density polyethylene, linear low density polyethylene, and ultra low density polyethylene. Examples of absorbent articles in which the absorbent binder composition may be used include diapers, diaper pants, training pants, feminine hygiene products, incontinence products, swimwear garments, and the like.

The absorbent binder composition of the invention is made by polymerizing monoethylenically unsaturated monomers, one of which contains an alkoxysilane functionality as defined in claim 1. The polymerization may be induced by a variety of initiation techniques including thermal initiation, radiation initiation, or redox chemical reactions. Various types of effective radiation initiation include ultraviolet, microwave, and electron-beam radiation. The initiator generates free radicals to cause polymerization of the monomers. The resultant copolymer includes latent moisture-induced crosslinking capability by incorporation of the alkoxysilane functionality. This copolymer may be applied, in a flowable state, to a substrate or other end use application. Moisture-induced crosslinking may be accomplished through hydrolysis of the alkoxysilane and subsequent condensation upon removal of the solvent from the substrate, either by evaporation of the solvent from the substrate or using any other effective technique. Alternatively, the hydrolysis of the alkoxysilane and subsequent condensation may occur after solvent removal by exposure of the coating to moisture in ambient air.

For instance, the absorbent binder composition of the invention can be made by combining a first aqueous monomer solution including a reducing polymerization initiator with a second aqueous monomer solution including an oxidizing polymerization initiator wherein the initiators react to form a binder composition. The polymerization of the

monomer solutions to form an absorbent binder composition may be carried out in about 100 minutes or less. The first aqueous monomer solution includes a monoethylenically unsaturated monomer such as a carboxylic acid salt, a sulphonic acid salt, a phosphonic acid salt or a combination thereof and an ethylenically unsaturated monomer including an acrylate or a methacrylate that contains an alkoxysilane functionality. The second aqueous monomer solution includes a monoethylenically unsaturated monomer such as a carboxylic acid salt, a sulphonic acid salt, a phosphonic acid salt or a combination thereof. Crosslinking of the resulting binder composition may be induced by concentrating the combined monomer solutions through the removal of water to promote condensation of silanols generated by the hydrolysis of the alkoxysilanes.

In another embodiment, an absorbent binder composition of the invention may be made by combining a first aqueous solution including a reducing polymerization initiator, a monoethylenically unsaturated monomer such as a carboxylic acid, a sulphonic acid, a phosphonic acid or a combination thereof and an ethylenically unsaturated monomer including an acrylate or a methacrylate that contains an alkoxysilane functionality with a second aqueous solution including an oxidizing polymerization initiator and a monoethylenically unsaturated monomer such as a carboxylic acid, a sulphonic acid, a phosphonic acid or a combination thereof. An amount of a basic material effective to at least partially neutralize the monoethylenically unsaturated monomer may be added to the first monomer solution and/or the second monomer solution.

The flexible absorbent binder composition described above can be prepared using a template polymerization process by which the monoethyleniclly unsaturated polymer and acrylate or methacrylate ester are polymerized in the presence of a pre-formed template polymer, which is the polyolefin glycol and/or polyolefin oxide. The polymerization can be carried out by reacting two different monoethylenically unsaturated monomers, one of which contains an alkoxysilane functionality. The polymerization may be induced by heat, radiation, redox chemical reactions, and other techniques. Suitable radiation initiators include without limitation ultraviolet, microwave, and electron beam radiation. The initiator generates free radicals to cause copolymerization of the monomers. In one embodiment, the polymerization reaction is carried out in an organic solvent such as ethanol. The polymerization may also occur in an aqueous solution, or in a combined aqueous and organic solvent.

The polyolefin glycol and/or oxide may or may not be graft polymerized onto the acrylate or methacrylate units during the polymerization process. The flexible absorbent binder composition may contain the polyolefin glycol and/or oxide as a separate component, or as part of the copolymer, or a combination of both.

The flexible absorbent binder composition has latent moisture-induced crosslinking capability due to the alkoxysilane functionality. The polymer composition may be applied, in a flowable state, to a substrate or other end use application. Moisture-induced crosslinking may be accomplished through hydrolysis of the alkoxysilane and subsequent condensation upon removal of the solvent from the substrate, either by evaporation of the solvent from the substrate or using any other effective technique. Alternatively, the hydrolysis of the alkoxysilane and subsequent condensation may occur after solvent removal by exposure of the coating to moisture in ambient air.

This invention is also directed to a combination including the absorbent or flexible absorbent binder composition applied to various substrates to provide an absorbent binder coating. The absorbent binder coating, which includes the hydrophilic polymer capable of post-application, moisture-induced crosslinking, is durable, relatively inexpensive to produce, and can be removed from surfaces and discarded after use.

The crosslinkable absorbent binder coating (including the flexible absorbent binder composition) can be directly applied to a person's skin, or to an article of clothing, or other suitable substrate. After use, the absorbent binder coating can be removed and discarded. Alternatively, a mesh layer may be placed between the substrate and the absorbent binder coating to facilitate removal of the absorbent binder coating after use. The absorbent binder coating may include a fragrance additive, odor-absorbing particles, and/or an antimicrobial additive to provide additional benefits.

Other substrates to which the absorbent binder coating may be applied include a mirror, camera lens, electronic display, or other types of surfaces that may be subjected to moist or humid conditions. The absorbent binder coating would absorb such moisture and thereby prevent fogging or other moisture-related occlusion of display surfaces. Other articles to which the absorbent binder coating may be applied include personal care absorbent garments, with the coating applied to a waistband or side panels to absorb moisture. A number of medical devices could also benefit from the application of the absorbent binder coating, including catheters and guide wires, as well as wound dressings.

In addition to providing absorbency and the ability to attach itself to substrates without the need for an additional binder, the absorbent binder coating can also be used to bond one layer to another. For example, the absorbent binder coating may bond a liquid-permeable layer to a liquid-impermeable layer, thereby providing some absorbency between the two layers, such as in an absorbent garment, or in a mattress pad.

Another application in which the absorbent binder coating can be used is in packaging material. For example, the absorbent binder coating can be applied to food storage containers, such as carry-out boxers, or produce storage bags, or shipping containers, to absorb excess moisture from the contents within the packaging as well as to release controlled water vapor to maintain optimum humidity.

Yet another application in which the absorbent binder coating can be used is in horticultural products. More

particularly, the absorbent binder coating can include one or more additives, such as fertilizer, auxin, fungicide, and/or floral preservative, and can be used to coat seeds or fertilizer pellets. Additionally, seeds or fertilizer pellets coated with the absorbent binder coating may be applied to a fabric, tape, or other substrate to provide for automated planting. The absorbent binder coating, with one or more additives, may be applied to plant cuttings for plant propagation or for cut flower preservation.

The present invention is also directed to an absorbent structure having a flexible absorbent binder provided at predetermined selected locations on a substrate to provide various advantageous properties. The flexible absorbent binder may be provided (e.g., formed) at predetermined selected locations on an absorbent core, another absorbent structure, or a carrier sheet or substrate, to provide controlled liquid intake, distribution, absorption, flexibility, and/or stiffness to the absorbent structure.

The flexible absorbent binder is formed from a precursor absorbent binder composition which is the flexible absorbent binder composition described herein. The precursor composition is applied to selected locations on the absorbent structure, and then crosslinked to form the flexible absorbent binder. The flexible absorbent binder is an absorbent or superabsorbent polymer which firmly adheres to the selected locations on the absorbent structure because it is crosslinked and formed directly on the structure. The absorbent structure may be an absorbent core of a personal care article, such as a diaper, diaper pant, sanitary napkin, swimwear or adult incontinence garment Absorbent cores are generally formed of absorbent materials such as cellulose fluff, wood pulp and the like. The absorbent structure may also be a layer of an absorbent article which is otherwise nonabsorbent, to which absorbency is imparted by inclusion of the flexible absorbent binder.

The absorbent binder composition (useful to form the flexible absorbent binder polymer) includes a hydrophilic polymer which is capable of post-application, moisture-induced crosslinking, is relatively inexpensive to produce, and has a high level of flexibility. The flexible nature of the flexible absorbent binder is useful when the binder is employed in personal care absorbent articles, as well as in other products which must be flexible and/or conformable to the wearer's body.

This invention is also directed to a three-layer absorbent structure and to absorbent articles containing it. The three-layer absorbent structure includes the following layers, in sequence, with no additional adhesive or other layers in between them:

a) a liquid-permeable fluid intake layer,
b) a flexible absorbent binder layer, and
c) a support layer.

The flexible absorbent binder layer serves as a fluid storage (absorbent) layer and also bonds the fluid intake layer to the support layer. The absorbent article may contain additional layers, so long as the above three layers of the absorbent structure occur in sequence. In some absorbent articles, the three-layer absorbent structure may not be accompanied by additional layers.

The three-layer absorbent structure is formed by applying an absorbent binder composition to one or both facing surfaces of the fluid intake layer and the support layer, bringing the fluid intake layer and support layer together, and crosslinking the absorbent binder composition to form the flexible absorbent binder layer. Because the flexible absorbent binder layer is formed (crosslinked) while the absorbent binder composition is in contact with the other layers, the flexible absorbent binder layer serves both as an absorbent layer and an adhesive (binder) layer in the three-layer absorbent structure, eliminating the need for additional adhesive or other bonding steps. The absorbent binder composition can be any of the absorbent or flexible absorbent binder compositions described herein.

With the foregoing in mind, it is a feature and advantage of the invention to provide an absorbent binder composition, a method of making such an absorbent binder composition, and absorbent articles which include the absorbent binder composition.

## BRIEF DESCRIPTION OF THE DRAWINGS

These and other objects and features of this invention will be better understood from the following detailed description taken in conjunction with the drawings, wherein:

Fig. 1 illustrates an absorbent binder coating applied to a substrate with a mesh layer between the coating and the substrate.
Fig. 2 illustrates a person's skin to which an absorbent binder coating is applied.
Fig. 3 illustrates a shoe to which an absorbent binder coating is applied.
Fig. 4 illustrates an undergarment to which an absorbent binder coating is applied.
Fig. 5 illustrates a garment having an underarm area to which an absorbent binder coating is applied.

Fig. 6 illustrates a bra to which an absorbent binder coating is applied.

Fig. 7 illustrates a garment having a bib area to which an absorbent binder coating is applied.

Fig. 8 illustrates a personal care absorbent garment to which an absorbent binder coating is applied.

Fig. 9 illustrates a laminate including an absorbent binder coating.

Fig. 10 illustrates a mirror to which an absorbent binder coating is applied.

Fig. 11 illustrates a camera to which an absorbent binder coating is applied.

Fig. 12 illustrates a digital display to which an absorbent binder coating is applied.

Fig. 13 illustrates a catheter to which an absorbent binder coating is applied.

Fig. 14 illustrates a wire to which an absorbent binder coating is applied.

Fig. 15 illustrates a wound dressing to which an absorbent binder coating is applied.

Fig. 16 illustrates a packaging material to which an absorbent binder coating is applied.

Fig. 17 illustrates a seed tape including an absorbent binder coating.

Fig. 18 illustrates a cut flower preservative including an absorbent binder.

Fig. 19 is a partially cut away top view of an absorbent article, in this case a sanitary napkin.

Fig. 19A is a partially cut away bottom view of the absorbent article of Fig. 19.

Fig. 19B is a sectional view of the absorbent article of Fig. 19, taken along a lateral direction 24 through the longitudinal center of the article.

Fig. 19C is a sectional view of the absorbent article of Fig.19, taken along a longitudinal direction 22 through the lateral center of the article.

Figs. 20-22 are top views of absorbent structures, in this case absorbent cores useful in the sanitary napkin of Figs. 19-19C. The absorbent cores include flexible absorbent polymer formed in selected locations for controlled fluid intake, distribution and absorption.

Fig. 23 is a top view of an absorbent article, in this case a sanitary napkin, having a dam of flexible absorbent polymer formed around its periphery to prevent leakage of bodily fluid.

Figs. 24-30 are edge views of absorbent structures which can be used as absorbent cores or intake (surge) layers in absorbent articles. The absorbent structures are formed using various staggered arrangements of flexible absorbent polymer.

Fig. 31 is a top view of an absorbent structure in which flexible absorbent material is configured to form an interior channel system and a peripheral dam system.

Figs. 32-34 are top views of absorbent structures, for instance absorbent cores, on which flexible absorbent material is disposed in patterns resembling braces to control the resiliency and folding properties of the absorbent structures.

Fig. 35 is an edge view of the absorbent structure of Fig. 32, showing how the flexible absorbent polymer may influence the folding pattern.

Fig. 36 is a plan view of an absorbent structure of the invention having a simplified three-layer structure, partially cut away to expose the underlying layers.

DEFINITIONS

Within the context of this specification, each term or phrase below will include the following meaning or meanings.

"Back region" or "back end region" is the rearward one-third of the longitudinal length of an absorbent structure.

"Bib area" refers to an area of a garment typically on a front of a wearer, onto which food or beverages could potentially fall.

"Binder" includes materials which are capable of attaching themselves to a substrate or are capable of attaching other substances to a substrate.

"Crotch region" is the central one-third of the longitudinal length of an absorbent structure.

"Feminine hygiene products" include sanitary pads and napkins, as well as tampons and interlabial feminine hygiene products.

"Fluid" refers to a substance in the form of a liquid or gas at room temperature and atmospheric pressure.

"Front region" or "front end region" is the forward one-third of the longitudinal length of an absorbent structure.

"High density polyethylene (HDPE)" refers to a polyethylene having a density of about 0.95 $g/cm^3$ or greater.

"Knife over roll coating" refers to a process in which a knife is positioned, with a specified gap, above a substrate that is moving beneath the knife on a moving roll. In this manner, the knife spreads a specified thickness of coating material onto the substrate.

"Layer" when used in the singular can have the dual meaning of a single element or a plurality of elements.

"Linear low density polyethylene (LLDPE)" refers to polymers of ethylene and higher alpha-olefin comonomers such as $C_3$-$C_{12}$ comonomers, and combinations thereof, having a density of about 0.900 to 0.935 $g/cm^3$.

"Liquid-impermeable" when used to describe a layer or laminate means that liquid such as urine will not pass through the layer or laminate under ordinary use conditions in a direction generally perpendicular to the plane of the

layer or laminate at the point of liquid contact.

"Liquid-permeable" refers to a layer or laminate that is not liquid-impermeable.

"Low density polyethylene (LDPE)" refers to a polyethylene having a density between about 0.91 and about 0.925 g/$cm^3$.

"Modifying agent" refers to a substance that may be added to a composition to modify the physical properties of the composition, such as the color or texture of the composition.

"Monomer(s)" as used herein includes monomers, oligomers, polymers, mixtures of monomers, oligomers and/or polymers, and any reactive chemical species which are capable of copolymerization with monoethylenically unsaturated carboxylic, sulphonic or phosphonic acid or salts thereof.

"Nonwoven" or "nonwoven web" refers to materials and webs or material having a structure of individual fibers or filaments which are interlaid, but not in an identifiable manner as in a knitted fabric. The terms "fiber" and "filament" are used interchangeably. Nonwoven fabrics or webs have been formed from many processes such as, for example, meltblowing processes, spunbonding processes, air laying processes, and bonded carded web processes. The basis weight of nonwoven fabrics is usually expressed in ounces of material per square yard (osy) or grams per square meter (gsm) and the fiber diameters are usually expressed in microns. (Note that to convert from osy to gsm, multiply osy by 33.91.)

"Personal care product" includes diapers, diaper pants, training pants, swim wear, absorbent underpants, adult incontinence products, feminine hygiene products, and the like.

"Roll printing" or "roll coating" refers to a process in which the application of a deposited material, generally as a paste, onto a substrate is carried out by transferring the deposited material from a roll onto the substrate in a more or less uniform layer using one or more rolls, which may be engraved, and a pool cylinder. A doctor blade is used to scrape any excess deposited material from the rolls or substrate. The doctor blade may be flat or have a patterned edge such as slots or ridges.

"Rotary screen printing" or "rotary screen coating" refers to a process that is a combination of roll printing or coating and screen printing or coating.

"Screen printing" or "screen coating" refers to a method of applying a deposited material by forcing the material to be deposited through a screen that may have uniform openings or patterned openings.

"Stranded composites" refer to sheets of material to which strands of an elastomeric material are adhered to create an elastomeric composite.

"Superabsorbent" refers to a water-swellable, water-insoluble organic or inorganic material capable, under the most favorable conditions, of absorbing at least about 10 times its own weight, or at least about 15 times its own weight, or at least about 20 times its own weight, or at least about 25 times its own weight in an aqueous solution containing 0.9 weight percent sodium chloride. The superabsorbent materials can be natural, synthetic, and modified natural polymers and materials. In addition, the superabsorbent materials can be inorganic materials, such as silica gels, or organic compounds such as cross-linked polymers.

"Ultra low density polyethylene (ULDPE)" refers to polymers of ethylene and higher alpha-olefin comonomers such as $C_3$-$C_{12}$ comonomers, and combinations thereof, having a density of about 0.860 to less than 0.900 g/$cm^3$.

"Unit" or "polymer unit" refers to a monomer or polymer portion of a copolymer molecule or blend component that includes a different molecular structure, compared to another portion of the copolymer or blend component

"Wound dressing" is used interchangeably with "bandage" and "dressing," and refers to a covering to be placed over a wound.

DESCRIPTION OF PREFERRED EMBODIMENTS

The present invention is directed to an absorbent binder composition as defined in claim 1 that includes a hydrophilic polymer having the capability of post-application, moisture-induced crosslinking. The present invention also includes a method of making and applying such an absorbent binder composition, and absorbent articles which utilize the absorbent binder composition. The absorbent binder composition can provide fluid retention properties in addition to adhesive properties. Thus, the absorbent binder composition is particularly suitable for use in forming absorbent products.

The present invention is also directed to articles which utilize the flexible absorbent binder composition.

The absorbent binder composition of the invention provides a water-soluble ionic polymer capable of sufficient spontaneous crosslinking within 10 minutes, at a temperature of 120°C or less, to reach an absorbent capacity of at least one gram of fluid per gram of absorbent binder composition, suitably at least three grams of fluid per gram of absorbent binder composition, using the centrifuge retention capacity test described herein. The term "spontaneous" crosslinking refers to crosslinking which occurs without radiation, catalysis, or any other inducement other than the specified temperature of 120°C or less, suitably 100°C or less. Eliminating the need for radiative crosslinking provides a significant processing advantage. The crosslinking at temperatures of 120°C or less, suitably 100°C or less, permits

the flexible absorbent binder composition to be applied to a substrate such as an absorbent article, and then crosslinked without degrading or damaging the substrate. The crosslinking occurs within 10 minutes, suitably within 8 minutes, particularly within 6 minutes provides an efficient, commercially feasible, cost-effective crosslinking process. The ionic polymer may bear a positive charge, a negative charge, or a combination of both, and has an ionic unit content of 15 mole percent or greater. The ionic polymer may include a variety of monomer units described above, and suitably contains a carboxyl group-containing unit or a quaternary ammonium-containing unit.

The monoethylenically unsaturated monomers that may be included in the binder composition include carboxyl group-containing monomers: monoethylenically unsaturated mono or poly-carboxylic acids, such as (meth)acrylic acid (meaning acrylic acid or methacrylic acid; similar notations are used hereinafter), maleic acid, fumaric acid, crotonic acid, sorbic acid, itaconic acid, and cinnamic acid;

Carboxylic acid salt-containing monomers: water-soluble salts (alkali metal salts, ammonium salts, amine salts, etc.) of monoethylenically unsaturated mono- or poly-carboxylic acids (such as sodium (meth)acrylate, trimethylamine (meth)acrylate, triethanolamine (meth)acrylate), sodium maleate, methylamine maleate;

Sulfonic acid group-containing monomers: aliphatic or aromatic vinyl sulfonic acids (such as vinylsulfonic acid, allyl sulfonic acid, vinyltoluenesulfonic acid, stryrene sulfonic acid), (meth)acrylic sulfonic acids [such as sulfopropyl (meth)acrylate, 2-hydroxy-3-(meth)acryloxy propyl sulfonic acid];

Sulfonic acid salt group-containing monomers: alkali metal salts, ammonium salts, amine salts of sulfonic acid group containing monomers as mentioned above; and/or

Quaternary ammonium salts.

The trialkoxy silane functional group has the following structure:

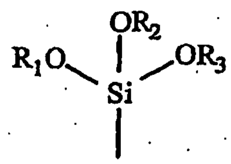

wherein $R_1$, $R_2$ and $R_3$ are alkyl groups independently having from 1 to 6 carbon atoms. The term "monomer(s)" as used herein includes monomers, oligomers, polymers, mixtures of monomers, oligomers and/or polymers, and any other reactive chemical species which is capable of co-polymerization with monoethylenically unsaturated carboxylic, sulphonic or phosphonic acid or salts thereof, quaternary ammonium salts. Acrylate or methacrylate ester monomers containing a trialkoxy silane functional group are used for this invention A particularly desirable monomer containing a trialkoxy silane functional group is methacryloxypropyl trimethoxy silane, commercially available from Dow Corning, having offices in Midland, Michigan, under the trade designation Z-6030 Silane. Other suitable monomers containing a trialkoxy silane functional group include, but are not limited to, methacryloxyethyl trimethoxy silane, methacryloxypropyl triethoxy silane, methacryloxypropyl tripropoxy silane, acryloxypropylmethyl dimethoxy silane, 3-acryloxypropyl trimethoxy silane, 3-methacryloxypropylmethyl diethoxy silane, 3-methacryloxypropylmethyl dimethoxy silane, and 3-methacryloxypropyl tris(methoxyethoxy) silane.

The amount of organic monomer having trialkoxy silane functional groups relative to the weight of the polymeric binder composition ranges from 0.1 to 20 weight percent. Suitably, the amount of monomer should exceed 0.1 weight percent in order provide sufficient crosslinking upon exposure to moisture. Typically, the monomer addition levels are between 0.1% and 15% of the weight of the polymeric binder composition; particularly, between 1.0% and 10% of the weight of the polymeric binder composition; or between 1.5% and 5.5% of the weight of the polymeric binder composition for some intended uses.

Optionally, the polymeric binder may include long chain, hydrophilic monoethylenically unsaturated esters, such as poly(ethylene glycol) methacrylate having from 1 to 13 ethylene glycol units. The hydrophilic monoethylenically unsaturated esters have the following structure: R=H or $CH_3$

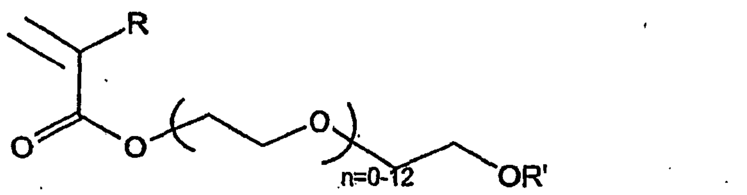

R'= H, alkyl, phenyl

The amount of monoethylenically unsaturated hydrophilic esters relative to the weight of the polymeric binder composition thereof may range from about 0 to about 75 weight percent of monomer to the weight of the polymeric binder composition. Typically, the monomer addition levels are between about 10% and about 60% of the weight of the polymeric binder composition; particularly, between about 20% and about 50% of the weight of the polymeric binder composition; or between about 30% and about 40% of the weight of the polymeric binder composition for some intended uses.

The polymeric binder composition may be prepared by adding a solution of the above monomers to an initiator solution, at a suitable temperature to generate free radicals, for example between about 50 and about 90 degrees Celsius. An initiator solution may be prepared by dissolving an initiator in a solvent. Possible solvents include, but are not limited to, alcohols such as ethanol. A variety of initiators may be useful in the practice of this invention. The polymerization initiator may be activated using a variety of methods including, but not limited to, thermal energy, ultraviolet light, redox chemical reactions. A suitable class of initiators are organic peroxides and azo compounds, with benzoyl peroxide and azobisisobutyronitrile (AIBN) as examples.

Compounds containing an O-O, S-S, or N=N bond may be used as thermal initiators. Compounds containing O-O bonds; i.e., peroxides, are commonly used as initiators for polymerization. Such commonly used peroxide initiators include: alkyl, dialkyl, diaryl and arylalkyl peroxides such as cumyl peroxide, t-butyl peroxide, di-t-butyl peroxide, dicumyl peroxide, cumyl butyl peroxide, 1,1-di-t-butyl proxy-3,5,5-trimethylcyclohexane, 2,5-dimethyl-2,5-di(t-butylperoxy)hexane, 2,5-dimethyl-2,5-bis(t-butylperoxy)hexyne-3 and bis(a-t-butyl peroxyisopropylbenzene); acyl peroxides such as acetyl peroxides and benzoyl peroxides; hydroperoxides such as cumyl hydroperoxide, t-butyl hydroperoxide, p-methane hydroperoxide, pinane hydroperoxide and cumene hydroperoxide; peresters or peroxyesters such as t-butylperoxypivalate, t-butyl peroctoate, t-butyl perbenzoate, 2,5-dimethylhexyl-2,5-di(perbenzoate) and t-butyl di(perphthalate); alkylsulfonyl peroxides; dialkyl peroxymonocarbonates; dialkyl peroxydicarbonates; diperoxyketals; ketone peroxides such as cyclohexanone peroxide and methyl ethyl ketone peroxide. Additionally, azo compounds such as 2,2'-azobisisobutyronitrile abbreviated as AIBN, 2,2'-azobis(2,4-dimethylpentanenitrile) and 1,1'-azobis(cyclohexanecarbonitrile) may be used as the initiator.

The method for making the absorbent binder composition can be carried out in a single step wherein polymerization and neutralization of the absorbent binder composition is achieved. The polymerization/neutralization reaction is conducted in an aqueous medium thereby eliminating the need for organic solvents.

More specifically, the absorbent binder composition can be made by combining a first aqueous monomer solution including a reducing polymerization initiator with a second aqueous monomer solution including an oxidizing polymerization initiator, wherein the initiators react to form a binder composition. The first aqueous monomer solution further includes a monoethylenically unsaturated monomer according to claim 1 and acrylate or methacrylate monomer that contains an alkoxysilane functionality. The second aqueous monomer solution includes a monoethylenically unsaturated monomer according to claim 1. Suitably, the binder composition is formed in about 100 minutes or less, or 60 minutes or less, desirably in about 30 minutes or less, or about 15 minutes or less, or in one embodiment about 10 minutes or less. The first and second aqueous monomer solutions may include any of the monoethylenically unsaturated monomers listed above.

In another embodiment, the first and/or the second aqueous solution may include a monoethylenically unsaturated monomer such, as a carboxylic acid, a sulphonic acid, a phosphonic acid or a combination thereof that may be at least partially neutralized or converted to the salt form *in situ.* In this embodiment, an amount of a basic material, such as sodium hydroxide, effective to at least partially neutralize the monoethylenically unsaturated monomer may be included in the first and/or second aqueous solution. Alternatively, the monoethylenically unsaturated monomer may be added to a basic solution such as, for example, a sodium hydroxide solution to form an aqueous monomer solution. Desirably, the monoethylenically unsaturated monomer in the first and/or second aqueous monomer solution is neutralized to provide a solution pH of about 5 to about 8 prior to polymerization with the acrylate or methacrylate monomer.

Suitably, the pH of the first and/or second aqueous monomer solution is adjusted to about 6.5 to about 7.0. The pH of the first aqueous solution may be adjusted prior to the addition of the ethylenically unsaturated monomer. Desirably, the pH of the first aqueous monomer solution is adjusted prior to the addition of the reducing polymerization initiator. The pH of the second aqueous solution may be adjusted prior to the addition of the oxidizing polymerization initiator. Alternatively, the pH of the combined first and second aqueous monomer solutions may be adjusted to about 6.5 to about 7.0.

The first aqueous monomer solution also includes an organic monomer capable of co-polymerization with monoethylenically unsaturated carboxylic, sulphonic or phosphonic acid or salts thereof, or quaternary ammonium salts. The above-described organic monomers that contain a trialkoxy silane functional group are used in the practice of this invention.

The first aqueous monomer solution may include the acrylate or methacrylate ester monomer containing a trialkoxy silane functional group in any suitable proportion to provide an absorbent binder composition including 0.1 to 20 composition weight percent of this monomer. Suitably, the amount of acrylate or methacrylate ester monomer containing a trialkoxy silane functional group should exceed 0.1 composition weight percent in order to provide sufficient crosslinking upon removal of water. Typically, the monomer addition levels are between 0.1 and 15 composition weight percent; particularly, between 1.0 and 10 composition weight percent; or between 1.5 and 5.5 composition weight percent for some intended uses.

In one embodiment, a surfactant may be added to the first and/or second aqueous monomer solution to disperse the ethylenically unsaturated monomer. One surfactant suitable for use in the present invention is a dioctyl sodium sulfosuccinate available under the trademark AEROSOL OT from Cytec Industries, Inc. of Paterson, New Jersey.

The first aqueous monomer solution further includes a reducing polymerization initiator. Suitable reducing polymerization initiators include, but are not limited to, ascorbic acid, alkali metal sulfites, alkali metal bisulfites, ammonium sulfite, ammonium bisulfite, alkali metal hydrogen sulfite, ferrous metal salts such as ferrous sulfates, sugars, aldehydes, primary and secondary alcohols, and combinations thereof. In one embodiment, the reducing polymerization initiator includes ascorbic acid.

The second aqueous monomer solution further includes an oxidizing polymerization initiator. Suitable oxidizing initiators include, but are not limited to, hydrogen peroxide, alkali metal persulfates, ammonium persulfate, alkylhydroperoxides, peresters, diacryl peroxides, silver salts, and combinations thereof. In one embodiment, the oxidizing polymerization initiator includes hydrogen peroxide.

Generally, when the first aqueous monomer solution is combined with the second aqueous monomer solution the reducing polymerization initiator reacts with the oxidizing polymerization initiator, e.g. a redox reaction, thereby initiating a polymerization reaction to form a binder composition including a monoethylenically unsaturated monomer and an ethylenically unsaturated monomer that has post-application, moisture-induced crosslinking capability.

The binder composition may be applied to a substrate and subsequently dried to form a cast film. Once the binder composition is applied to the substrate, crosslinking can be moisture-induced by hydrolysis and condensation of alkoxysilanes. For example, crosslinking of the binder composition can be induced by concentrating the binder composition on the substrate through the removal of the water to promote condensation of silanols generated by hydrolysis of alkoxysilanes. Typically, crosslinking begins at a solution concentration above about 30 percent by weight binder composition.

Alternatively, the resulting binder composition may be applied to a substrate, such as for the purpose of adhering various components of an absorbent product to one another during the manufacturing process of absorbent products. In another embodiment, the binder composition may be applied to a substrate as a coating by itself, thereby serving as an absorbency additive. In either of these embodiments, the binder composition is suitably present in any concentration that provides a viscosity suitable for the application process. The binder composition may be applied to the substrate using any suitable application process, including knife over roll coating, or roll coating, either in a continuous coverage or a patterned coverage . Printing applications are other suitable application techniques, including gravure printing, screen, and jet printing. The binder composition may also be applied to the substrate using a spray application,

In another embodiment, the absorbent binder composition may be prepared using a continuous process wherein the polymerization and/or neutralization reaction is carried out in a suitable reactor that conveys the resulting binder composition, upon completion of the polymerization reaction, directly to an apparatus for applying the absorbent binder composition onto the substrate. Such a continuous process may be desirable where conditions, such as high heat, may cause premature crosslinking of the binder composition that would hinder application of the absorbent binder composition onto the substrate.

In addition, modifying agents such as compatible polymers, plasticizers, colorants, and preservatives may be incorporated in the binder composition.

For some intended uses the polymeric binder composition of this invention provides very flexible coatings and should therefore have a glass transition temperature below about 30 degrees Celsius, or below about 10 degrees Celsius, as measured by Differential Scanning Calorimetry (DSC), and a bending modulus lower than the substrate to which they are applied. Suitably, the absorbent binder composition, in combination with the substrate, has a Gurley stiffness value of less than 320 milligrams (mg), or less than 160 mg, or less than 60 mg. Suitable substrates to which the binder composition may be applied include, but are not limited to, nonwoven, woven, and knitted fabrics; cellulosic tissue sheets; plastic films, including polypropylene, low density polyethylene, high density polyethylene, linear low density polyethylene, and ultra low density polyethylene; LYCRA stranded composites; and elastomer net composites.

Furthermore, crosslinked films of the polymeric binder composition are capable of absorbing at least 80 percent of their dry weight of 0.9% saline solution with no load applied during swelling. The absorbency of polymeric binder composition may range from about 80 percent of their dry weight of 0.9% saline solution to about 4000 percent of their dry weight of 0.9% saline solution with no load applied during swelling. Typical absorbency with no load applied during swelling is from 80% to 1500% of dry weight, particularly, between about 100% and about 900%; or between about 300% and about 700% of dry weight for some intended uses.

The absorbent binder composition can be used in the manufacture of absorbent products, thereby adding absorbent capacity to such absorbent products. Examples of such articles include training pants, diapers, diaper pants,

feminine hygiene products, swimwear, incontinence products, absorbent toweling, other personal care or health care garments, including medical garments, or the like. As used herein, the term "incontinence products" includes absorbent underwear for children, absorbent garments for children or young adults with special needs such as autistic children or others with bladder/bowel control problems as a result of physical disabilities, as well as absorbent garments for incontinent older adults.

As indicated above, the invention is directed to a flexible absorbent binder composition. The flexible absorbent binder composition includes 15 to 99.8% by mass of monoethylenically unsaturated polymer units, suitably 25 to 89.5% by mass, particularly 30 to 79% by mass, or 50 to 70% by mass. The monoethylenically unsaturated polymer units are monoethylenically unsaturated carboxylic acid units and salts thereof, monoethylenically unsaturated sulphonic acid units and salts thereof, and monoethylenically unsaturated phosphonic acid units and salts thereof, and monoethylenically unsaturated quaternary ammonium salts. Suitable monoethylenically unsaturated monomers that can be used to form the monoethylenically unsaturated polymer units include without limitation any of the monoethylenically unsaturated monomers described above.

The flexible absorbent binder composition also includes 0.1 to 20% by mass of polyacrylate ester units, such as acrylate and/or methacrylate ester units, that include an alkoxysilane functionality. The acrylate and/or methacrylate ester units are copolymerized with the monoethylenically unsaturated monomer units. In particular, the absorbent binder composition may include 0.5 to 15% by mass of the acrylate and/or methacrylate ester units, for instance 1.0 to 10% by mass, for instance 1.5 to 5.5% by mass.

As described above, the alkoxysilane functionality is a functional group or moiety that reacts with water to form a silanol group. One suitable alkoxysilane group is the above-described trialkoxy silane group having the following structure:

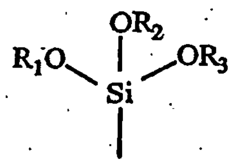

wherein $R_1$, $R_2$ and $R_3$ are alkyl groups independently having from 1 to 6 carbon atoms. Any of the monomers described above, which contain the trialkoxysilane functional group, are suitable.

The flexible absorbent binder composition also includes 0.1 to 75% by mass polyolefin glycol and/or polyolefin oxide units, suitably 5 to 75% by mass, particularly 10 to 60% by mass, particularly 20 to 50% by mass, particularly 30 to 40% by mass. The polyolefin glycol or oxide may be a glycol or oxide of an olefin polymer having 2 to 4 carbon atoms. Polyethylene glycol, polyethylene oxide, polypropylene glycol and polypropylene oxide are examples of suitable polymer units. The polyolefin glycol and/or polyolefin oxide may include on average 30 to 15,000 glycol and/or oxide units per molecule. The weight average molecular weight of polyolefin glycol units may range from 200 to 8000. When polyolefin oxide units are employed, they may have a weight average molecular weight of 100,000 to 600,000.

Polyolefin glycols and polyolefin oxides are commercially available, and are common. To prepare the flexible absorbent binder composition of the invention, a pre-formed polyolefin glycol and/or oxide may be dissolved or dispersed in a reaction vessel which includes an aqueous solvent or carrier, an organic solvent or carrier such as ethanol, or a miscible combination of aqueous and organic solvent or carrier. The monomers used to form the monoethylenically unsaturated polymer units and the polyacrylate ester units are added to the solution and polymerized using a template polymerization process in which the polyolefin glycol or oxide serves as a template polymer. Before initiation, the polar groups of the monomers, for instance the acid groups of acrylic acid, are attracted to the polyolefin glycol and/or polyolefin oxide through hydrogen bonding. The steric alignment of the monomers, with the polyolefin glycol and/or oxide serving as backbone, aids in the polymerization and typically increases the chain length of the polymerizing unit. During the polymerization, radical polymerizing chains may become attached to the template polymer, resulting in grafting of polyolefin glycol and/or oxide to the copolymer being formed. However, this graft polymerization need not occur. The resulting flexible absorbent binder composition includes the polyolefin glycol and/or oxide attached to, and/or blended with, the copolymer of the monoethylenically unsaturated polymer units and the acrylate or methacrylate ester units that include the alkoxysilane functionality.

The polymerization may be initiated using a variety of methods, including without limitation thermal energy, ultraviolet light, and redox chemical reactions. A solution of the above ingredients may be added to an initiator solution at a temperature suitable for generating free radicals, for instance about 50 to about 90° C. An initiator may be prepared by dissolving an initiator in an organic or aqueous solvent. A suitable class of initiators are organic peroxides and azo compounds, with benzoyl peroxide and azobisisobutylnitrile (ABN) as examples.

Compounds containing an O-O, S-S, or N=N bond may be used as thermal initiators. Any of the above-described

thermal initiators can be used when preparing the flexible absorbent binder composition.

Alternatively, redox initiation can be used to prepare the flexible absorbent binder composition, as described above. The polyolefin glycol and/or polyolefin oxide may be added to the first monomer solution, or the second monomer solution, or both. Surfactants and other ingredients can also be added, as described above.

In one embodiment used to prepare the flexible absorbent' binder composition, the monoethylenically unsaturated polymer unit is a cationic polymer. The cationic polymer is advantageous because it provides a) inherent antimicrobial properties, b) enhanced attraction and retention into cellulose fibers in a suspension, and c) enhanced attraction to superabsorbent particles which are negatively charged. Suitable cationic polymers include those prepared by copolymerizing a monomer 1) selected from a) acryloyloxyethyl-trialkyl-substituted ammonium salts, b) acryloyloxypropyl-trialkyl-substituted ammonium salts, c) acrylamidoethyl-trialkyl-substituted ammonium salts, and d) acrylamidopropyl-trialkyl-substituted ammonium salts, with a monomer 2) selected from a) methacryl esters which contain an alkoxysilane group capable of moisture-induced crosslinking and b) acryl esters which contain an alkoxysilane group capable of moisture-induced crosslinking. Other monomers may also be present, for instance, an acrylic acid or acrylamide. The polymerization is conducted in the presence of a polyolefin glycol and/or polyolefin oxide as described above, suitably a polyethylene glycol. The cationic monoethylenically unsaturated monomer unit and the polyolefin glycol are present in the amounts described above.

The cationic monoethylenically unsaturated polymer may be prepared by a redox initiation process, according to the following reaction. The cationic copolymer is then coated and dried onto a substrate to form the crosslinked flexible absorbent coating.

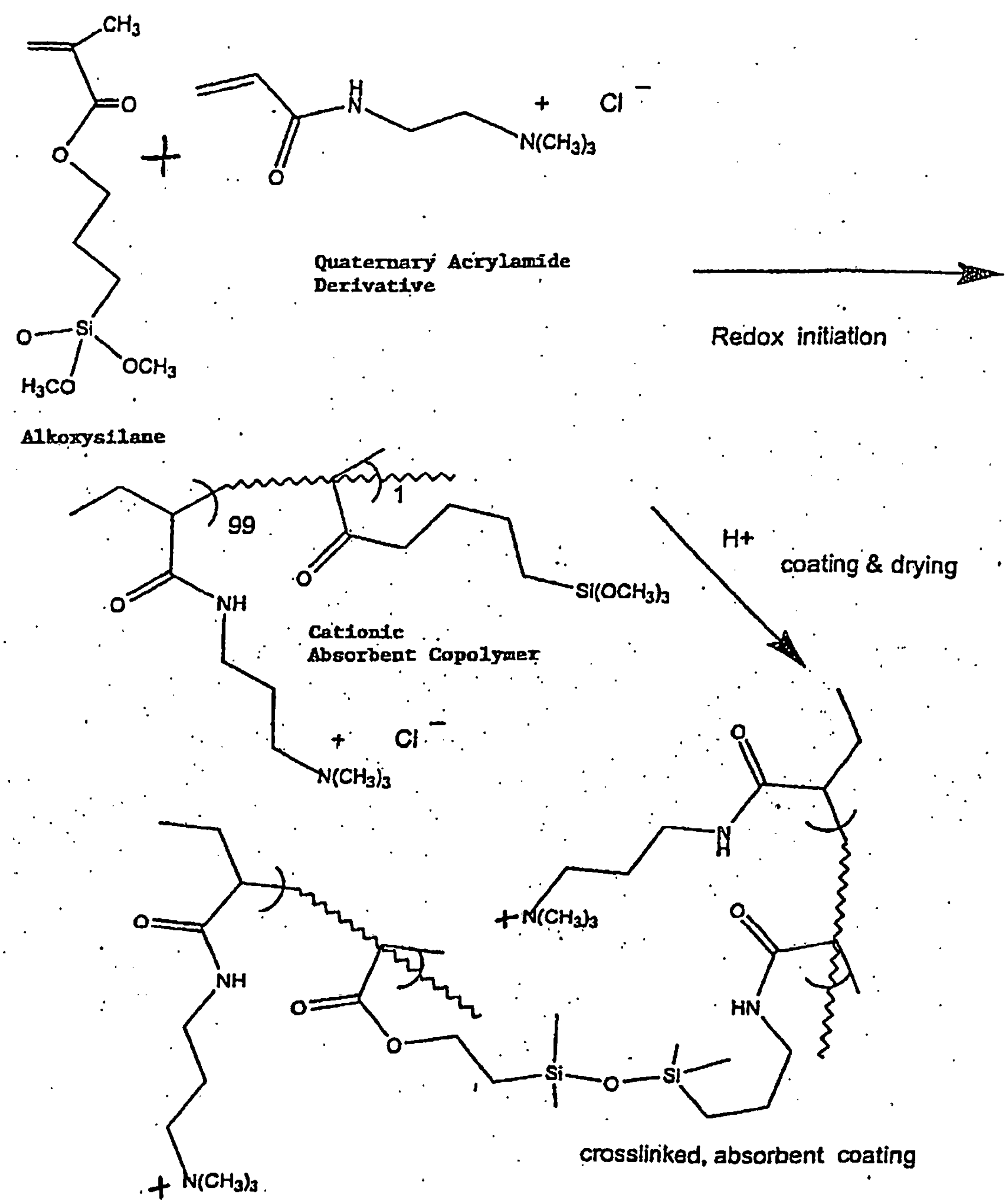


Applications where the cationic flexible absorbent binder composition is useful include without limitation fabric and foam coatings for gauze and wound dressings. The cationic flexible absorbent binder composition adheres to the substrate and wound, and helps contain antimicrobial and healing agents. The cationic flexible absorbent binder composition may also form a lubricious, antimicrobial coating for a catheter or guide wire. Additionally, the cationic flexible absorbent binder composition may be used to coat cellulose tissue or paper in a wide variety of applications ranging from absorbent paper towels to cartons for carrying hot food. The cationic absorbent binder composition may also be provided in a spray or roll-on form for use as a deodorant.

The flexible absorbent binder composition maybe applied to a substrate and subsequently dried to form a cast film. Once the absorbent binder composition is applied to the substrate, crosslinking can be moisture-induced by hydrolysis and condensation of alkoxysilanes. For example, crosslinking of the flexible absorbent binder composition can be induced by concentrating the absorbent binder composition on the substrate through the removal of the water to promote condensation of silanols generated by hydrolysis of alkoxysilanes. Typically, crosslinking begins at a solution concentration of about 30 percent or greater by weight absorbent binder composition. Furthermore, if the substrate material has hydroxyl group functionality on its surface, then the silanols within the binder composition may react with the hydroxyl groups to form a covalent bond between the binder and the hydroxyl-containing surface. Non-limiting

examples of substrates with hydroxyl surface functionality include glass, sand and cellulose.

Alternatively, the flexible absorbent binder composition may be applied to a substrate, such as for the purpose of adhering various components of an absorbent article to one another during the manufacturing process of absorbent articles. Absorbent articles include without limitation any of the personal care absorbent articles and medical absorbent articles described above. In another embodiment, the flexible absorbent binder composition may be applied to a substrate as a coating by itself, thereby serving as an absorbency additive. In either of these embodiments, the flexible absorbent binder composition is suitably present in any concentration that provides a viscosity suitable for the application process. The flexible absorbent binder composition may be applied to the substrate using any suitable application process, including knife over roll coating, or roll coating, either in a continuous coverage or a patterned coverage. Printing applications are other suitable application techniques, including gravure printing, screen, and jet printing. The binder composition may also be applied to the substrate using a spray application.

In another embodiment, the flexible absorbent binder composition may be prepared using a continuous process wherein the polymerization and/or neutralization reaction is carried out in a suitable reactor that conveys the flexible absorbent binder composition, upon completion of the polymerization reaction, directly to an apparatus for applying the absorbent binder composition onto the substrate. Such a continuous process may be desirable where conditions, such as high heat, may cause premature crosslinking of the flexible absorbent binder composition that would hinder application of the absorbent binder composition onto the substrate.

The present invention is also directed to a combination of a substrate and a crosslinkable absorbent binder coating. The term "absorbent binder coating" includes crosslinkable coatings formed from the absorbent binder composition or the flexible absorbent binder composition described above, suitably from the flexible absorbent binder composition.

In this aspect of the invention, a substrate is at least partially coated with the crosslinkable absorbent binder coating, which forms an absorbent and lubricious hydrogel upon exposure to aqueous solutions. Also, upon exposure to water, the coating may release various additives, as described in greater detail below. The crosslinkable absorbent binder coating, particularly in a fast-drying alcohol solution, provides a convenient means of applying absorbent capacity to a wide range of surfaces. Application of the absorbent binder coating, followed by evaporation of a carrier solvent within the coating, results in formation of a coating capable of absorbing aqueous fluid or water vapor. Because the coated articles are capable of rapid absorption of water vapor, these articles are particularly suitable for humidity control products. The coating may be applied to a substrate using any suitable application process, including knife over roll coating, or roll coating, either in a continuous coverage or a discontinuous or patterned coverage. Printing applications or other suitable application techniques, including gravure printing, screen, and jet printing. The coating may also be applied to the substrate using a spray application. Suitable substrates to which the coating may be applied include, but are not limited to, nonwoven, woven, and knitted fabrics; cellulosic tissue sheets; plastic films, including polypropylene, low density polyethylene, high density polyethylene, linear low density polyethylene, and ultra low density polyethylene; LYCRA stranded composites; elastomer net composites; various articles of clothing; a person's skin; non-porous surfaces; foam material; seeds; and fertilizer pellets.

Referring to Figs. 1-8, the crosslinkable absorbent binder coating may be used as a self-applying treatment, with the coating applied directly to various substrates, such as clothing or a person's skin, to absorb sweat, spills, or other forms of wetness. The coating may be temporarily applied to such surfaces, and subsequently may be removed by peeling the coating off of the surface or washing the coating off of the surface without causing permanent damage to the underlying surface. Additionally, a mesh layer 24, such as mesh gauze, may be placed between the substrate 22 and the coating 20 to facilitate peel removal, as shown in Fig. 1.

As shown in Fig. 2, for example, the absorbent binder coating 20 can be applied to a person's underarm skin surface 26, in the form of a roll-on or spray application, thereby serving as an antiperspirant. Additives may be included in the coating, particularly in an alcohol solution of the coating. Such additives may include a fragrance additive, antimicrobial additive, and/or odor-absorbing particles, such as nanoparticle silica, available from Nissan Chemicals America, Houston, Texas, as 20% wt/wt aqueous suspension under the trade designation SNOWTEX-C. In this manner, the deodorant provides wetness protection from the absorbent film and odor protection from the odor-absorbing particles suspended in the absorbent film. Alternatively, or additionally, the cationic absorbent coating, as described in the Example below, may have inherent antimicrobial properties to further inhibit the generation of malodorous compounds that result from bacterial action. The absorbent binder coating may also be applied to other areas of the body including feet, back, face, or any other suitable skin surface.

As shown in Figs. 3-8, for example, the absorbent binder coating 20 can be applied to various types of clothing such as shoes 28 or shoe inserts 30 (Fig. 3) or undergarments 32 (Fig. 4), including disposable undergarments, to provide entrapment of fluid while maintaining the comfort and fit of the clothing. Other examples of clothing to which the coating may be applied include shirts 34 and other garments having an underarm area 36 (Fig. 5) to capture sweat, bras 38 (Fig. 6) to capture milk when nursing, as well as treatment of any clothing having a bib area 40 (Fig. 7) so that the coating may serve as a spray-on or painted-on bib.

The absorbent binder coating 20 can also be applied to personal care absorbent garments, such as training pants, diapers, diaper pants, feminine hygiene products, swimwear, incontinence products, other personal care or health care garments, including medical garments, or the like. For illustrative purposes, a personal care absorbent undergarment, namely a training pant 42, is shown in Fig. 8.

As shown in Fig. 8, a training pant 42 having permanently bonded sides, or a training pant having refastenable sides in a fastened position, defines a three-dimensional pant configuration having a waist opening 50 and a pair of leg openings 52. The absorbent binder coating 20 can be applied to a waistband 44 of the garment around the waist opening to provide additional absorbency in the garment.

The training pant 42 includes a body side liner 46 which is configured to contact the wearer, and an outer cover 48 opposite the body side liner which is configured to contact the wearer's clothing. An absorbent assembly (not shown) is positioned or located between the outer cover 48 and the body side liner 46. Other materials that are designed primarily to receive, temporarily store, and/or transport liquid along the mutually facing surface with the absorbent assembly can also be incorporated into the absorbent article, thereby maximizing the overall absorbent capacity of the absorbent assembly, if desired. One suitable material is referred to as a surge layer (not shown) and includes a material having a basis weight of about 50 to about 120 grams per square meter (gsm), such as a through-air-bonded-carded web of a homogenous blend of 60 percent 3.3dTex (3 denier) type T-256 bicomponent fiber including a polyester core/polyethylene sheath and 40 percent 6.7dTex (6 denier) type T-295 polyester fiber, both commercially available from Kosa Corporation of Salisbury, North Carolina, U.S.A. Another example of a suitable surge layer may include a material made of 6.7dTex (6 denier) polyethylene terephthalate (PET) and 6.7dTex (6 denier) bicomponent binder fiber, having a basis weight of about 50 to about 120 gsm. The absorbent binder coating 20 can be applied to the surge material to provide an additional reservoir for excess fluid that runs off the absorbent assembly. Discontinuous application of the absorbent binder coating 20 can be used to direct the flow of fluid to particular portions of the absorbent assembly since uncoated portions of the surge material will have a faster rate of fluid transfer than the coated portions. Conversely, coated portions of the surge material provide restriction of flow from the absorbent assembly back into the surge material, thereby inhibiting "rewet" of the body side liner 46.

The outer cover 48 desirably includes a material that is substantially liquid-impermeable, and can be elastic, stretchable or nonstretchable. The outer cover 48 can be a single layer of liquid-impermeable material, but desirably includes a multi-layered laminate structure in which at least one of the layers is liquid-impermeable. For instance, the outer cover 48 can include a vapor-permeable layer and a liquid-impermeable layer that are suitably joined together by the absorbent binder coating 20. As another example, the absorbent binder coating 20 can be used to join together the outer cover 48 and the body side liner 46. Alternatively, or additionally, the absorbent binder coating 20 can be applied to the outer cover 48 to provide a reservoir to capture any liquid run-off.

In order to forestall leakage around the leg openings 52, the garment may include a pair of containment flaps (not shown) which are configured to provide a barrier to the transverse flow of body exudates. The elasticized containment flaps define an unattached edge which assumes an upright, generally perpendicular configuration in at least a crotch region of the training pant 42 to form a seal against the wearer's body. The absorbent binder coating 20 can be applied to the flap material to further forestall leakage around the leg openings 52. Suitable constructions and arrangements for the containment flaps are generally well known to those skilled in the art and are described in U.S. Patent 4,704,116 issued November 3,1987 to Enloe.

As shown in Fig. 8, the training pant 42 may include a pair of transversely opposed front side panels 54, and a pair of transversely opposed back side panels 56. The side panels 54, 56 may be integrally formed with the outer cover 48 and/or the body side liner 46, or may include two or more separate elements. Suitable materials for side panels, as well as processes of incorporating side panels into a training pant, are known and are described, for example, in U.S. Patent 4,940,464 issued July 10,1990 to Van Gompel et al. In one embodiment, the absorbent binder coating 20 may be applied to an inner surface of the side panels 54, 56 to provide additional absorbency in the garment.

The absorbent binder coating can be applied to any suitable surface or combination of surfaces of the personal care absorbent garment. The coating provides for enhanced liquid absorbent capacity for a portion of the material that comes into contact with liquid. Portions of the coated material that do not come into contact with liquid are capable of absorbing water vapor which is readily released from absorbents including swollen superabsorbent and/or fluff pulp. The ability to trap excess water vapor provides for reduced relative humidity within the garment. Reduced humidity is well known to provide improved skin health.

In addition to forming laminates of layers within a personal care absorbent garment, other laminates 58 may also be formed with the absorbent binder coating 20. For example, the coating may be applied between a liquid-permeable substrate 60, such as a nonwoven web, and a liquid-impermeable substrate 62, such as a film, to form a laminate 58, as shown in Fig. 9. Such a laminate is particularly suitable for use in a personal care absorbent garment, as mentioned, as well as in a mattress pad. The coating between the two layers bonds the layers together as well as provides absorbency within the middle of the laminate. More particularly, the absorbent coating absorbs water vapor penetrating through a liquid-impermeable breathable film layer and traps the water vapor to prevent or reduce dampness from escaping to a

liquid-permeable nonwoven web layer.

The absorbent binder coating 20 may be used as a component for use as part of a surgical drape (such as reinforcing fabric surrounding a fenestration or along an edge of a drape), and for inclusion in protective apparel, such as a surgical gown, where the nonwoven web side is positioned towards the wearer's skin while the film side is positioned away from the skin in order to absorb body perspiration or where the nonwoven faces external to the protective garment to contain fluid drips, splashes, and the like, that may otherwise run downwards and off the garment. The absorbent coating may absorb condensed water vapor between the film and the nonwoven when water vapor transmission is slowed during penetration through the liquid-impermeable film.

The absorbent binder coating 20 may also be applied to the surface of other articles, particularly those susceptible to fogging, such as mirrors 64 (Fig. 10), endoscopic cameras or other cameras 66 (Fig. 11), electronic displays 68 (Fig. 12) or other display materials, to provide a hydrophilic and absorbent surface that minimizes the formation of water droplets on the surface, thereby inhibiting fogging. Another example of a suitable application for the absorbent binder coating 20 is to a windowsill and/or window frame to prevent mold from growing due to a change in humidity during the winter. Additionally, the absorbent binder coating 20 may be applied to a catheter 70 (Fig. 13) or other medical devices, a guide wire 72 (Fig. 14), or other wire, thereby forming a lubricious coating on the object with a potential for covalent bonding to the surface. Compared to other coatings, the absorbent binder coating is applied by a low-cost process with no gamma radiation or UV treatment required.

In another embodiment, the absorbent binder coating 20 may be applied to a wound dressing 74, as shown in Fig. 15. More particularly, the coating 20 may be applied to gauze or other woven material included in the wound dressing, thereby providing absorbency and high integrity while preventing the material from adhering to any wounds. Alternatively, the wound dressing may include a nonwoven, film, and/or foam substrate to which the coating 20 is applied, resulting in a high-absorbency wound dressing that does not adhere to wounds. Another feature of the absorbent binder coating 20 is that it can act as a reservoir for releasable components that are to be delivered from the coating 20 to a region outside of the coating 20, such as, for example, a wound. In this embodiment, the coating can act to provide a controlled release of such compounds as wound healing agents, therapeutic agents, bioactive agents, antibiotics, bactericides, fungicides, drugs, growth factors, peptides, proteins, enzymes, emollients, antiseptics, anti-oxidants, wetting agents, and mixtures thereof. Suitable wound healing agents may include, for example, chitosan, niacinamide ascorbate, or the chitosan niacinamide ascorbate salt.

In still another embodiment, the absorbent binder coating 20 may be applied to a packaging material substrate to form a packaging material having absorbent properties. The substrate may be a plastic film, carton stock, or foam, for example. One example of a coated packaging material 76 is illustrated in Fig. 16. More particularly, the coating 20 may be applied to an inside surface of a food storage container, shipping container, or any other suitable storage container, or filler material such as bubble wrap or STYROFOAM® foam pellets, to provide a surface capable of absorbing excess water vapor into the coating. This capability prevents the release of water droplets formed from vapor condensation. Containers for take-out hot food, for example, typically trap the warm, moist air radiating from the food, causing the food to become soggy as a result of evaporation and condensation of water that accumulates on the inner surface of the container and drips into the food. The coating 20 applied to the inner surface could help keep take-out food from becoming soggy. As another example, containers for shipping produce often accumulate moisture which causes the produce to spoil. By applying the coating 20 to an inside surface of containers for shipping produce, the coated containers would have an "anti-dew" property that keeps liquid water off the produce and reduces the chances for spoilage. Similarly, the coating 20 can be applied to produce bags to maintain optimum humidity and to keep liquid water off the produce, thereby keeping the produce from getting soggy and extending the freshness.

In yet another embodiment, the absorbent binder coating 20 can be used to form various horticultural products. For example, the coating 20 can be applied to seeds to help retain seed moisture and viability. Additionally, fertilizer can be included in the coating 20, particularly in a coating polymer solution, either as a solution of fertilizer or a dispersion of fertilizer particulate. The coating 20 with the fertilizer additive can be applied to seeds to help retain seed moisture and viability, as well as to slowly release the fertilizer into a growing medium once the seeds are planted and watered.

As another horticultural example, illustrated in Fig. 17, seeds 78 covered with the absorbent binder coating 20 can be applied to a fabric or tape 80 or other suitable substrate. In this manner the coating 20 also functions as an adhesive to secure the seeds 78 to the substrate 80, which allows for automated planting. Upon application of water, the seeds 78 are released from the substrate 80 and the coating 20 helps retain seed moisture and viability, while fertilizer within the coating 20, if present, is slowly released into the growing medium once the seeds 78 are watered.

The absorbent binder coating 20 may also be applied as a polymer solution to fertilizer pellets for time-released fertilization. With each watering, additional fertilizer is released into the growing medium, with the amount of fertilizer released being proportional to the amount of water applied. Alternatively, the coating solution may also function as a bonding agent to fabricate fertilizer pellets from smaller fertilizer particles.

Other additives, instead of or in addition to fertilizer, may be included in the absorbent binder coating 20. These additives may include a fungicide to combat rotting, auxin or other root growth-stimulating hormone, and/or floral preservative 82. Auxin is often applied to stem cuttings in a powder form, which is difficult to distribute in an even manner. The coating 20 including auxin, a floral preservative 82, and/or other additives, may be in a liquid or gel form to allow for uniform application to stem cuttings 84, as shown, for example, in Fig. 18. Uniform application of such additives as auxin, for example, can lead to more uniform root production for plant propagation. A mild fungicide in the coating 20 could prevent rotting of the cuttings before adventitious roots emerge.

The absorbent binder coating 20, including fungicide, fertilizer, and/or floral preservative, may be applied to freshly cut wounds on flower stems to preserve freshly cut flowers. More particularly, the coating 20 helps retain existing moisture within the stems and also distributes beneficial additives when the stems are submerged in water.

The invention is also directed to an absorbent structure having a flexible absorbent binder provided at predetermined selected locations on a substrate. The flexible absorbent binder can be formed using any of the absorbent or flexible absorbent binder compositions described above, and is desirably formed using a flexible absorbent binder composition.

Disposable absorbent articles such as, for example, many of the feminine care absorbent products, can include a liquid pervious topsheet, a substantially liquid impervious backsheet joined to the topsheet, and an absorbent core positioned and held between the topsheet and the backsheet. The topsheet is operatively permeable to the liquids that are intended to be held or stored by the absorbent article, and the backsheet may be substantially impermeable or otherwise operatively impermeable to the intended liquids. The absorbent article may also include other components, such as liquid wicking layers, liquid intake layers, liquid distribution layers, transfer layers, barrier layers, and the like, as well as combinations thereof. Disposable absorbent articles and the components thereof can operate to provide a body-facing surface and a garment-facing surface. As used herein, the body-facing or bodyside surface means that surface of the article or component which is intended to be disposed toward or placed adjacent to the body of the wearer during ordinary use, while the outward, outward-facing or garment-side surface is on the opposite side, and is intended to be disposed to face away from the wearer's body during ordinary use. Such outward surface may be arranged to face toward or placed adjacent to the wearer's undergarments when the absorbent article is worn.

Figs. 19 through 19C illustrate an example of a suitable article, such as the representatively shown feminine care article. Referring to Fig. 19, the feminine care article can, for example, be a feminine care pad or napkin 120, and the article can have a lengthwise longitudinal direction 122, a transverse, laterally extending cross-direction 124, first and second longitudinally opposed end portions 272 and 272a, and an intermediate portion 276 located between the end portions. As representatively shown, the longitudinal dimension of the article is relatively larger than the lateral dimension of the article. The article 120 can include a topsheet or cover 126, a baffle 128, and an absorbent structure 130 positioned between the cover and baffle. The absorbent structure 130 can at least include an intake layer 132 and a shaping or absorbent layer 136.

Referring to Fig. 19, the absorbent article 120, in this case a sanitary napkin, may embody one or more of the absorbent structures of the invention. In the lower section of Fig. 19 layers, respectively, of the article 120 of the invention have in part been cut out to show the layers below. The lowermost layer (outer cover or baffle) of the article 120 is formed by a liquid-impermeable layer 128. The liquid-impermeable layer 128 can be made of a polypropylene film, or instance. The liquid-impermeable layer 128 serves as so-called garment-protecting layer which prevents liquid which has penetrated into the absorbent article and which is retained therein from escaping downwards from the absorbent body. This prevents the wearer's undergarment from being stained. The liquid-impermeable layer 128, which is referred to synonymously as an outer cover or baffle, can be breathable to water vapor.

Referring to Fig. 19A, the absorbent article 120 also includes two laterally extending, inward folding wings 242 and 242a, hook fastening materials 246 and 246a attached to inner surfaces of end regions of the wings, respectively, and loop fastening materials 248 and 248a attached to or forming part of outer surfaces of the user's end regions of the wings. When the wings 242 and 242a are folded inward as shown, over a wearer's garment, the hook and loop fastening regions overlap and engage each other to secure the absorbent article 120 in place. Adhesive bands 138 can be used to secure the baffle 128 to a peelable release layer 240. When the release layer 240 is removed (peeled away), the bands 138 of adhesive provide additional securement of the absorbent article 120 to an inner surface of the wearer's garment.

Figs. 19B and 19C illustrate exploded sectional views of the absorbent article 120, shown in the lateral direction 224 (Fig. 19B) and in the longitudinal direction 222 (Fig. 19C). As illustrated, the topsheet 126 and intake layer 132 are adhered together by a first adhesive layer 226. The intake layer 132 and absorbent layer 136 are adhered together by a second adhesive layer 232. The absorbent layer 136 and baffle 128 are adhered together by a third adhesive layer 236. The wings 242 and 242a may be bound at manufacturer's ends thereof to the baffle 128 by adhesive bands 228.

Additional absorbent structures, namely feminine care pad designs, are described in U.S. Patent Application 10/379,942, filed on 04 March 2003, entitled "Perimeter Embossing In An Absorbent Article," and in U.S. Patent Application 10/392,116, filed on 19 March 2003, entitled "Multilayer Absorbent Article." These documents are incorporated by reference.

Figs. 20-22 illustrate various embodiments of absorbent structure 130 in which a flexible absorbent binder,

formed at selected locations designated by shaded regions, serves as or replaces the intake layer 132 of the absorbent structure 130. The flexible absorbent binder layer 132 may be formed at selected locations on the absorbent layer 136, which may have a conventional absorbent construction as shown. Alternatively, in some embodiments, the absorbent layer 136 may be omitted, and the flexible absorbent binder layer 132 may be selectively formed directly on the liquid impermeable outer cover of the absorbent article and may provide suitable absorbent capacity. In each of Figs. 20-22, the flexible absorbent binder layer 132 is formed at selected locations to provide channels therebetween which facilitate liquid intake, distribution and absorption by the absorbent core 115 or into the flexible absorbent binder layer 132.

Referring to Fig. 20, the flexible absorbent binder layer 132 forms a dam 122 around the outer periphery of absorbent structure 130, to facilitate peripheral absorption and alleviate leakage of fluid from around the periphery. Additionally, the flexible absorbent binder occupies a plurality of locations 124 inward from the dam 122. The inward locations 124 of flexible absorbent binder, and the dam 122, define a network' 117 of channels which are devoid of flexible absorbent binder, and which facilitate liquid intake and distribution. The channel network 117 includes a relatively wide central channel 111 longitudinally bisecting a central region 131 of the absorbent structure 130, and four interconnecting narrower channels 113 surrounding the central region 131. The channel network 117 also includes a plurality of radially projecting channels 119 in a front region 133 and a back region 135 of the absorbent structure 130. The channels 119 become gradually wider as they approach the dam 122.

Fig. 21 illustrates another embodiment of absorbent structure 130 in which the flexible absorbent binder layer 132 covers a substantial portion of the front and back regions 133 and 135, and the peripheral portions of the central region 131. Openings in the flexible absorbent binder layer 132 define channels 137 in the front and back regions 133 and 135. Channels 137 are wider toward the central region 131, and become progressively narrower away from the central region, with each channel 137 terminating at a point in the front or back region 133 or 135. The flexible absorbent binder layer 132 also defines a rectangular area 140 in the central region 131. The channels 137 and the small openings 142 of the rectangular area 140 may permit liquid entering the absorbent structure to quickly pass directly to the lower absorbent layer 136 of the absorbent structure 130, or to the impermeable baffle 128 if a lower storage layer is not used. The absorbent layer 136 or impermeable baffle 128 may laterally distribute some of the liquid and pass it up to the flexible absorbent binder layer 132.

In the absorbent structure 130 of Fig. 22, the flexible absorbent binder layer 120 includes only a plurality of elongated rectangles 126 extending longitudinally in and through the central region 131. The lower absorbent layer 136 is exposed substantially throughout the front and back regions 133 and 135, near the peripheral edges of the central region 131, and in channels 139 formed between the rectangles 126 in the central region. In the absorbent core of Fig. 22, much of the liquid would be absorbed by the rectangles 126 of flexible absorbent binder, causing swelling and widening of the rectangles 126.

In each of the embodiments of Figs. 20-23, the channels defined by voids in the flexible absorbent binder layer 132 serve the dual purposes of a) facilitating liquid distribution, and b) permitting expansion of the flexible absorbent binder when it becomes wet By providing room for the flexible absorbent binder to expand, gel blocking is alleviated. In various embodiments, the lower absorbent layer 136 may be composed entirely of absorbent fibers such as wood pulp or cellulose fluff, or may include additional particles or fibers of superabsorbent polymer or enhanced absorption. In some instances, the flexible absorbent binder layer may be present without a lower storage layer. Fig. 23 illustrates an embodiment of absorbent structure 130 in which the flexible absorbent binder layer 120 is employed only as a peripheral dam 122. In this embodiments, it may be advantageous to mix superabsorbent particles or fibers with the cellulose fluff in lower storage layer 114, for enhanced absorption.

Superabsorbent particles or fibers can also be mixed with and incorporated into the flexible absorbent binder or bound to its surface. A modifying agent, such as a menses modifying agent, can also be combined with the flexible absorbent binder. These additives improve the retention functionality of the structure and provide desired product absorbent capacity to reduce leakage. The increased functionality can diminish the need for a lower absorbent layer 136.

Figs. 24-30 are edge views of absorbent structures which incorporate the flexible absorbent binder in a variety of layered configurations to enhance fluid intake and absorption and alleviate gel blocking. In each instance, the flexible absorbent binder may be applied in rectangles or stripes, for instance, as in Fig. 22. However, unlike Fig. 22, the flexible absorbent binder in Figs. 24-30 is applied in two or more layers. Also, the absorbent structures shown are not necessarily limited to absorbent cores, but may be used as surge layers, cover material layers, other intake layers, or lower storage layers in absorbent articles. The stripes, rectangles or the like of flexible absorbent binder are shown in edge view in Figs. 24-30.

Referring to Fig. 24, the absorbent structure 150 includes three layers 152, 154 and 156 of flexible absorbent binder. The layers 152 and 154 are formed on first and second sides of a liquid pervious layer which may be a nonwoven material or an intake layer, for instance, a bonded carded web. The layer 156 is formed on a support layer 157 which, depending on the position of structure 150 in an absorbent article, may be another liquid permeable (e.g., intake) layer or a liquid impermeable (e.g., outer cover) layer.

Each of the flexible absorbent binder layers 152, 154 and 156 includes a plurality of rectangles 155 of flexible

absorbent binder, with spaces 159 between the rectangles. The rectangles 155 are spaced apart within each layer and are offset (staggered) in the adjacent layers, so that a) the rectangles 155 of flexible absorbent binder may expand when wet, without blocking fluid flow, and b) fluid may flow between the rectangles 155 from one of the layers 152, 154, 156 to the next. Both of these features facilitate fluid flow and absorption, and alleviate, gel blocking. The fluid can quickly migrate to the support layer 157 for absorption or distribution along the length of the product. The upper layers 152 and 154 are available for additional retention capacity with increased fluid loadings. The rectangles 155 maybe replaced with other shapes of flexible absorbent binder, so long as the flexible absorbent binder in each layer is applied at a plurality of locations, desirably in stripes, with spaces between the locations. In the embodiment shown, the rectangles are configured to provide a vertically continuous dam 151 of flexible absorbent binder at the peripheral edges of the absorbent structure.

The absorbent structure 150 of Fig. 25 resembles the one shown in Fig. 24, except that there are only two layers 152 and 154 of flexible absorbent binder, formed on opposing sides of a liquid permeable layer 153. The stripes or rectangles 155 of flexible absorbent binder are spaced apart and staggered in much the same fashion as in the upper layers 152,154 in Fig. 24.

The absorbent structure 150 of Fig. 26 resembles the one shown in Fig. 24 except that the stripes or rectangles 155 of flexible absorbent binder are relatively narrower in the two upper layers 152, 154. Some of the rectangles 155 in the upper layer 152 are particularly narrow, with wide spaces 159 between them. The rectangles 155 in the middle layer 154 are relatively narrow in the absorbent structure of Fig. 26, compared to the absorbent structure of Fig. 24. The effect of using narrower stripes or rectangles 155 of flexible absorbent binder is that the absorbent structure 150 of Fig. 26 is more open, and facilitates more rapid fluid flow between the layers.

The absorbent structure 150 of Fig. 27 is very similar to the one shown in Fig. 26. The difference is that, in Fig. 27, there is no continuous peripheral dam 151 formed of flexible absorbent binder rectangles 155 in all three layers. The dam 151 is formed only in the middle and lower layers 154 and 156 of flexible absorbent polymer, and not in the upper layer 152.

In the absorbent structure 150 of Fig. 28, each of the layers 152, 154 and 156 of flexible absorbent binder is formed on a separate substrate layer. The substrate layer 153 supporting layer 154 of flexible absorbent binder, and the substrate layer 149 supporting layer 154 of flexible absorbent binder, are each liquid permeable intake layers, for instance, spunbond webs. The substrate layer 157 supporting layer 156 of flexible absorbent binder may be liquid-permeable layer or liquid impermeable, depending on where the absorbent structure 150 is positioned in an absorbent article. If the absorbent structure 150 is positioned as a cover layer facing a bodyside liner, or as a surge layer, or both, then the lower support layer 157 may be liquid-permeable, and may be positioned above an absorbent core. If the absorbent structure 150 is used essentially as an absorbent core, then the lower support layer 157 may be liquid-impermeable, for instance, an outer cover.

By having each of the flexible absorbent binder layers 152, 154 and 156 independently supported as shown in Fig. 28, they may be positioned so that they can move and shift relative to each other during use, without binding the flexible absorbent binder layers to each other. This arrangement further facilitates passage of liquid between the layers. Liquid transport and distribution are further enhanced, and gel blocking is further reduced. The offset flexible absorbent binder stripes in the absorbent structures described in Figs. 24-28 also allow the absorbent core to form an inverted "V" shape or W-fold geometry under lateral compression between the wearer's legs. An inverted "V" shape or W-fold geometry can also provide positive shaping of the layers for more comfortable fit, less bunching, and optimal contact of the flexible absorbent binder with the fluid source. Stripes of flexible absorbent binder can be configured for optimal comfort and fit as well as fluid distribution and storage.

Fig. 29 illustrates an absorbent article 160 in which the flexible absorbent binder layers 162, 164 and 166 are composed of spaced apart, semi-cylindrical or horseshoe-shaped members 165 of flexible absorbent binder. The members 165 in each layer are formed, respectively, on substrates 161, 163 and 167 which have a three-dimensional topography. Examples of three-dimensional substrates 161, 163 and 167 which are liquid permeable include creped nonwoven webs (e.g., spunbond webs), creped apertured thermoplastic films, and nonwoven webs and apertured films which have been embossed or otherwise molded to have a three-dimensional topography. The semi-cylindrical configuration of flexible absorbent binder members 165 results in relatively large open channels 169 between the adjacent layers of flexible absorbent polymer, and between the individual members 165 of a given layer.

The absorbent article 160 of Fig. 30 also includes three layers 162, 164 and 166 of semi-cylindrical flexible absorbent binder members 165, each formed on a separate three-dimensional substrate 161, 163 or 167. In the embodiment of Fig. 30, the layers 164 and 166 are inverted relative to each other and the space between them is filled with a layer 168 of conventional absorbent material, such as a layer including wood pulp or cellulose fluff. In this embodiment, the absorbent layers 164, 166 and 168 may together perform a primary storage function in an absorbent core, whereas the absorbent layer 162 may perform liquid receiving and distribution functions, and a secondary storage function.

Fig. 31 is a top view of an absorbent structure 170 which can include the three-dimensional flexible absorbent binder topography illustrated in any of Fig. 24-30. Absorbent structure 170 includes an illustrated substrate layer 161,

which is a generally liquid permeable three-dimensional substrate as described above. Semi-cylindrical flexible absorbent binder members 165 are oriented in the longitudinal direction of the absorbent structure in the central region 173, and are separated by channels 169. A large storage area 171 of flexible absorbent binder is provided in each end region 175 and 177 of the absorbent structure 170. A much narrower peripheral dam 174 is provided near the perimeter over the entire circumference of the absorbent structure 170. The absorbent structure 170 may be used in an absorbent article, for instance a feminine care pad, with the channels 169 serving to move amounts of the received fluid into both end regions for storage in the large storage areas 171. The peripheral dam 174 prevents fluid leakage from the sides and ends. The substrate 161 is typically liquid permeable, for instance, an absorbent layer of cellulose fluff. However, the substrate 161 may also be a liquid impermeable outer cover if it is desired to simplify the absorbent structure 170 and minimize its thickness.

Figs. 32-35 illustrate absorbent articles in which the flexible absorbent binder is applied at selected locations to control the stiffness, resiliency and folding properties of the article when under lateral compression from the user's legs. Referring to Fig. 32, an absorbent article 180, which can be a sanitary napkin, includes a wider chassis portion 182 including a bodyside liner and outer cover, for instance, and a narrower absorbent core 184. This absorbent article can be rectangular or shaped. A plurality of stripes 185 of flexible absorbent binder are positioned parallel to each other, overlying the core 184 in the lateral direction. The stripes 185 of flexible absorbent binder may be formed directly on the absorbent core 184, or may be formed on a liquid receiving layer above the core. The stripes 185 of flexible absorbent binder may also be formed on the liquid-impermeable outer cover (baffle) 128. In addition to providing enhanced absorbency, the stripes 185 of flexible absorbent binder act as braces which discourage lateral folding and bunching of the absorbent core, both during storage and during use on a wearer. The braces promote raising of the central region of the article into an inverted "V" shape to provide more intimate contact at points of fluid entry, resulting in less leakage and a more comfortable fit. This pattern can be combined with other patterns, such as described in Figs. 20-31

Fig. 33 illustrates, in plan view, an absorbent article similar to the one shown in Fig. 32 except for differences in the flexible absorbent binder stripes. In the absorbent article of Fig. 33, the stripes 185 of flexible absorbent binder are formed laterally in or over the absorbent core 184. The stripes 185 are laterally centered on or over the absorbent core, but do not have sufficient length in the lateral direction to completely traverse the absorbent core. Two longitudinal stripes 187 of flexible absorbent binder are formed along the lateral edges of the absorbent core, and extend the length of the absorbent article 180. The longitudinal stripes 187 can be formed on the impermeable outer cover, or underneath, in or on the absorbent core 184.

In addition to providing controlled absorption properties and fit, the distribution of flexible absorbent binder shown in Fig. 33 permits the article 180 to have a W-shaped lateral folding shown in edge view in Fig. 35. By applying an inward force at both lateral edges 181 and 183 of the absorbent article similar to the compressive forces of the user's legs, this W-shaped folding can be achieved. If the flexible absorbent binder were not selectively positioned as shown in Fig. 33, or in another helpful configuration, the W-shaped folding would be difficult to achieve. The W-shaped folding is useful to promote positive shaping of the layers for more comfortable fit, less bunching, and optimal contact of the flexible absorbent binder with the fluid source, leading to less leakage. This pattern can be combined with other patterns, such as described in Figs. 20-31.

Fig. 34 illustrates, in plan view, another embodiment of the absorbent article. In the embodiment of Fig. 34, four stripes 186 of flexible absorbent binder are formed in a diamond configuration in the crotch region of the absorbent article. The diamond-shaped arrangement of flexible absorbent binder provides both elevation ("lift") and increased absorbent capacity to the crotch region during wear. The diamond-shaped arrangement can be formed on the impermeable outer cover 128, or underneath, in or on the absorbent core 184. This arrangement also promotes W-shaped folding of the absorbent article during wear. This pattern can be combined with other patterns, such as described in Figs. 20-31.

Superabsorbent can play a role in each of the described patterns of flexible absorbent binder in Figs. 20-34. Retention can be increased and improved with the binding of superabsorbent onto the flexible absorbent binder. Additionally, modification of the menses with the use of menses modifying agents also bound to the flexible absorbent binder can also improve fluid movement and retention within the flexible absorbent binder.

This invention is also directed to a three-layer absorbent structure in which a flexible absorbent binder layer prepared using any of the absorbent or flexible absorbent binder compositions described above, is joined to both a fluid intake layer and a support layer. The flexible absorbent binder layer provides both absorbent and adhesive properties, thus reducing the number of layers required for an absorbent article.

As described above, the absorbent article of Figs. 19-19C includes multiple fluid receiving layers (topsheet 26, fluid intake layer 32) an absorbent layer 36, an outer cover or baffle 28, and multiple adhesive layers (126, 132 and 136) holding these layers together.

Fig. 36 illustrates an absorbent article 350 formed using the simplified three-layer absorbent structure 154 of the invention. The absorbent article 350 illustrated includes three layers, namely a liquid intake layer 360, a layer 356 of flexible absorbent binder, and a support layer 352. In the embodiment shown, the intake layer 360 is also a bodyside

liner, and the support layer 352 is an outer cover. The flexible absorbent binder layer 356 is directly joined to the adjacent functional layers 352 and 360, without an additional adhesive. This is accomplished by applying an absorbent binder composition to facing surfaces of one or both layers 352 and 360, bringing the layers 352 and 360 together so that the absorbent binder composition contacts both layers, and crosslinking the absorbent binder composition to form the flexible absorbent binder layer 356. The flexible absorbent binder is an absorbent or superabsorbent polymer formed using techniques described above.

Because the flexible absorbent binder layer 356 is in contact with layers 352 and 360 as it is being formed, the layer 356 adheres to layers 352 and 360 in addition to serving as an absorbent (fluid storage) layer. Thus, the absorbent structure 354 of the invention provides three layers bound together in sequence, namely a fluid receiving layer, a flexible absorbent binder layer, and a support layer, without intervening adhesive layers.

The flexible absorbent binder layer 356 maybe formed as a continuous layer having uniform thickness, or as a discontinuous or nonuniform layer which provides flow channels, liquid retention dams, or other desired attributes. However, because the flexible absorbent binder layer 356 is intended as a sole or primary absorbent layer in the simplified absorbent article, the flexible absorbent binder should be present in sufficient thickness and quantity, and over a sufficient area to provide substantially all of the liquid absorption capacity that is required by the end use application. Alternatively, superabsorbent particles can be incorporated into the flexible absorbent binder layer 356 to provide a portion of the liquid absorption capacity required by the end use application.

The absorbent article 350 may include only the three layers 352, 356 and 360 forming the absorbent structure 354, or may include additional layers on one or both sides of the absorbent structure 354 (but not between the layers 352, 356 and 360). In either case, the absorbent article 350 will have a simplified construction compared to conventional absorbent articles because a) the flexible absorbent binder layer 356 (with or without superabsorbent particles) provides essentially all of the required absorbent capacity, and b) the flexible absorbent binder layer 356 binds to the adjacent layers 352 and 360 without intervening adhesive layers.

Referring again to Fig. 36, the support layer 352 may be a liquid-impermeable outer cover material. Suitable outer cover materials include without limitation polyolefin films (e.g., films of polypropylene and polyethylene homopolymers and copolymers), breathable polyolefin films (e.g., stretch-thinned films formed from one or more polyolefins blended with calcium carbonate or other suitable particulate fillers), and laminates of a breathable polyolefin film and a polyolefin nonwoven web (e.g., a spunbond web). Alternatively, the absorbent article 350 maybe designed to include one or more functional layers, such as a dampness-inhibiting "spacer" structure, between the outer cover and the flexible absorbent polymer layer 356. In such instances, the support layer 352 may be any layer that is positioned directly below the flexible absorbent polymer layer 352 in the absorbent article 350. Depending on the application, the support layer 352 may be a nonwoven web, woven web, knitted fabric layer, cellulose layer, plastic film, plastic foam, staple fiber layer, elastomeric net composite, stranded composite or another suitable material.

The fluid-receiving layer 360 may be an apertured film, an open nonwoven layer such as a spunbond layer, bonded carded web or staple fiber web, an open-celled (e.g., reticulated) foam, a cellulose web, or any suitable open structure capable of receiving and/or distributing liquid. The fluid-receiving layer 360 may be homogeneous in the thickness direction or have a gradient structure. The desired composition of fluid receiving layer 360 may depend on whether the fluid-receiving layer 360 is used as a bodyside liner, or whether it is an interior fluid-receiving layer (e.g., a surge/transfer or compensation layer) used in addition to one or more other fluid receiving layers. In the simplest embodiment, the absorbent article 350 may include only the three-layer absorbent structure 354 composed of the fluid receiving layer 360, flexible absorbent binder layer 356 and support layer 352. In other embodiments, the three-layer absorbent structure 354 may form only a part of a more complex layer structure in an absorbent article 350.

## EXAMPLES

### Example 1

An initiator solution was prepared by dissolving 0.354 grams benzoyl peroxide in 300 milliliters of ethanol. The monomer solution was prepared by mixing 24.15 grams of acrylic acid (24 mass %), 73.5 grams of poly(ethylene glycol) methyl ether methacrylate (74 mass %) and 1.46 grams of 3-(trimethoxysilyl)propyl methacrylate (2 mass %) in 250 milliliters of ethanol. The initiator solution was heated in a jacketed reactor to 75 degrees Celsius with stirring. The monomer solution was added dropwise to the initiator solution. The polymerization solution was stirred and heated at 75 degrees Celsius for approximately 2 hours at which time a solution of 0.096 grams azobisisobutyronitrile (AIBN) in 30 ml ethanol was added. Stirring and heating at 75 degrees Celsius for an additional hour at which time a second solution of 0.096 grams AIBN in 30 ml ethanol was added to the polymerization solution. A third addition of 0.096 grams AIBN in 30 ml ethanol was made after one more hour at 75 degrees Celsius. Stirring and heating continued at 75 degrees Celsius for a total reaction time of about 7 hours. The reactor was cooled to 20 degrees Celsius and the solution was stirred under nitrogen atmosphere overnight. A portion of the polymer solution was dried for 16 hours at room temperature

to create a sticky, water-absorbent film. A portion of the dry polymer film weighing 0.25 grams was sealed in a tea bag and immersed in 0.9% saline for 60 minutes. The tea bag containing the swollen film was centrifuged for 3 minutes at 1600 rpm (equivalent to 3Gs) to remove free fluid. The polymer film had absorbency of 680% (6.8 grams) of 0.9 percent saline per gram of dry polymer film.

Example 2

A portion of the polymer solution from Example 1 was treated with sodium hydroxide solution to neutralize a portion (50%) of acrylic acid units in the binder polymer in order to increase the absorbency of the crosslinked film generated by drying the solution. The neutralization was done by adding 5.25 grams of an aqueous 48.5% sodium hydroxide solution to 236 grams of polymer solution (16.2 % polymer) and stirring at room temperature or 5 minutes. A portion of the polymer solution was dried or 16 hours at room temperature to create a water-absorbent film. A portion of the dry polymer film weighing 0.21 grams was sealed in a tea bag and immersed in 0.9% saline for 60 minutes. The tea bag containing the swollen film was centrifuged or 3 minutes at 1600 rpm (equivalent to 3Gs) to remove free fluid. The polymer film had absorbency of 650% (6.5 grams) of 0.9 percent saline per gram of dry polymer film.

Example 3

An initiator solution was prepared by dissolving 0.705 grams benzoyl peroxide in 300 milliliters of ethanol. The monomer solution was prepared by mixing 58.8 grams of acrylic acid (36 mass %), 100.8 grams of poly(ethylene glycol) methyl ether methacrylate (62 mass %) and 2.92 grams of 3-(trimethoxysilyl)propyl methacrylate (2 mass %) in 250 milliliters of ethanol. The initiator solution was heated in a jacketed reactor to 75 degrees Celsius with stirring. The monomer solution was added dropwise to the initiator solution. The polymerization solution was stirred and heated at 75 degrees Celsius for approximately 2 hours at which time a solution of 0.191 grams azobisisobutyronitrile (AIBN) in 30 ml ethanol was added. Stirring and heating at 75 degrees Celsius for an additional hour at which time a second solution of 0.191 grams AIBN in 30 ml ethanol was added to the polymerization solution. A third addition of 0.191 grams AIBN in 30 ml ethanol was made after one more hour at 75 degrees Celsius. Stirring and heating continued at 75 degrees Celsius for a total reaction time of about 7 hours.

Sodium hydroxide solution was used to neutralize a portion (70%) of the acrylic acid units in the binder polymer. Additional water was added to make a 10% binder solution with the solvent composition approximately 83% ethanol and 17% water.

A 9-cm by 34-cm piece of loft bonded carded web surge material made according to U.S. Patent No. 5,364,382, manufactured by Kimberly-Clark Corporation, with a basis weight of about 45 gsm and a density of 0.04 g/cm$^3$ measured at a pressure of 207 Pa (0.05 psi) was immersed in about 30 grams of the binder solution to thoroughly saturate the fabric. The dry weight of the fabric was 1.78 grams. Excess fluid was squeezed out, and the saturated surge sample was dried for 4 minutes at 105 degrees Celsius in a Mathis through-air-dryer oven, available from Werner Mathis in Palmer, Pennsylvania. After drying, the coated fabric weighed 5.71 grams, so 3.93 grams of absorbent binder were applied.

The coated surge material was tested for absorbency according to the Absorbency Under Load for Composites (AULC) which is described in the Test Methods section below. The surge material coated with the absorbent binder had absorbency of 9.4 g 207 Pag (0.03 psi) 8.7 g/g at 2.07 kpa (0.3 psi) and 8.2 g/g at 6.21 kpa (0.9 psi). Without the absorbent binder applied, the surge material has absorbency of less than 2 g/g since the fluid is substantially removed by vacuum, as described in the test method.

This example demonstrates that a fluid storage material can be made by applying the absorbent binder composition, by any suitable means, to a substrate.

Example 5

Two monomer solutions were prepared separately. Solution No. 1 was prepared as follows. To 14.4 grams (0.20 moles) of acrylic acid in a 200 ml beaker was added 33.3 grams of a 18% aqueous solution of polyethylene.glycol 8000, followed by a solution of 3.2 grams of sodium hydroxide in 21.4 grams of distilled water. Then, 0.18 grams (1.02 x $10^{-3}$ moles) of ascorbic acid was added to the solution. This mixture was stirred with a magnetic stir bar at about 60 rpm in a bath of water at about 23°C until the ascorbic acid was dissolved and the mixture cooled to 23°C.

Solution No. 2 was prepared in the following manner. To 14.4 grams (0.20 moles) of acrylic acid, in a 300 ml beaker was added to 33.3 grams of a 18% aqueous solution of polyethylene glycol 8000 (mol. wt. = 8000) followed by a solution of 3.2 grams of sodium hydroxide in 21.4 grams of distilled water, 0.57 ml of 30% aqueous hydrogen peroxide and 1.0 ml (5.42 x $10^{-3}$ moles) of 3-(trimethoxysilyl)propyl methacrylate. The ingredients were added with stirring to produce a clear solution. This mixture was stirred with a magnetic stir bar at about 60 rpm in a bath of water at about

23°C to provide a clear solution cooled to 23 °C.

A third solution was prepared by dissolving 8 grams (0.20 moles) sodium hydroxide in 160 grams of distilled water.

Solution No. 2 was added to Solution No. 1 while stirring with a magnetic stir bar at about 60 rpm. A thermocouple was used to monitor the temperature and observe the reaction exotherm. The polymerization reaction began within about 30 seconds of mixing as the temperature rose from 23°C to 40°C. A maximum temperature of about 70°C was observed after three minutes of mixing the two solutions. The polymerization transformed the combined solutions into a soft gel. The gel was cut into pieces of about 1 $cm^3$ and added to the solution of 8 grams (0.20 moles) sodium hydroxide in 160 grams of distilled water. With continued stirring, aided by an ULTRATURAX homogenizer at 11,000 rpm, the soft gel became a viscous translucent solution.

The resulting aqueous binder composition was cast into a film by pouring 25.1 grams of solution into a polystyrene weigh boat, available from VWR International, catalog no. 12577-027, with surface area of about 80 $cm^2$, and allowing the water to evaporate overnight in a hood at room temperature. The resulting film weighed 4.62 grams, indicating a solution concentration of about 18.4%.

The absorbent capacity of the film was tested using the Centrifuge Retention Capacity test described in the test method section. The film had an absorbent capacity of 12.2 g/g.

Example 6

An aqueous binder composition was prepared according to the procedure of Example 5. This example was used to determine the speed of crosslinking in the following manner. Six 10-cm by 10-cm pieces of loft bonded carded web surge material made according to U.S. Patent No. 5,364,382, by Kimberly-Clark Corporation, with a basis weight of about 45 gsm and a density of 0.04 g/$cm^3$ measured at a pressure of 345 Pa (0.05 psi) were each immersed in about 12 grams of the binder solution to thoroughly saturate the fabric. The dry weight of each fabric was weighed prior to saturation with the binder solution. The weights of the starting solution and of the residual solution were measured, with the difference being equal to the weight of solution applied to each piece of fabric. The saturated surge sample was placed on an aluminum screen and dried for times ranging from 5 to 15 minutes at 105°C in a Mathis through-air-dryer oven, available from Werner Mathis in Palmer, Pennsylvania. After drying, for the specified time, listed in Table 1, the coated fabric was weighed. After removal from the through-air-dryer oven the absorbent capacity of each of the test fabrics was determined using the Centrifuge Retention Test. The samples were then dried for 3 hours at 80°C in a Constant Temperature Oven, Model DK-63, available from Scientific Products. The re-dried weight was used to determine the fraction of the binder solution that was extractable. For instance, 100% extractable would be indicative of a fully soluble polymer with no crosslinking and no absorbent capacity. The extent of crosslinking is inversely proportional to the percent of extractable components.

These results (shown in Table 1) indicate crosslinking begins even before the coated fabric is fully dry. While not wishing to be bound by theory, the rate of crosslinking is believed to be a function of the concentration of the polymer in solution. Any means of rapidly removing the solvent, such as higher temperature, greater airflow, or lower pressure, will increase the polymer concentration and the rate of crosslinking.

**Table 1**

| Drying Time (minutes) | Fabric Weight (grams) | Weight of Solution Applied (grams) | Weight After Drying (grams) | g/g Absorbent Capacity Based On Weight After Drying | Re-dry Weight (grams) | % Extractable |
|---|---|---|---|---|---|---|
| 5 | .51 | 12.34 | 5.61 | 0.7 | .79 | 86 |
| 7 | .52 | 11.15 | 3.66 | 3.5 | .83 | 77 |
| 9 | .51 | 11.02 | 2.50 | 3.7 | .95 | 62 |
| 11 | .51 | 11.63 | 1.80 | 5.4 | 1.01 | 44 |
| 13 | .54 | 11.64 | 1.66 | 6.0 | 1.07 | 35 |
| 15 | .53 | 12.46 | 1.61 | 5.8 | 1.11 | 31 |

The percent extractable was calculated as:

$$100\% \times \frac{\text{(weight after drying minus weight after redrying)}}{\text{weight after drying}}$$

Example 7

This example relates to the preparation of a cationic flexible absorbent binder polymer. Two monomer solutions were prepared separately. Solution No. 1 was prepared as follows. To 55.0 grams of a 75% solution of (3-acrylamidopropyl) trimethyl ammonium chloride (0.20 moles) was added 21.3 grams of deionized water, and 6.0 grams of PEG 200 (molecular weight 200). Then, 0.18 grams (1.02 x $10^{-3}$ moles) of ascorbic acid was added to the solution. This mixture was stirred with a magnetic stir bar at about 60 rpm in a bath of water at about 23°C until the ascorbic acid was dissolved and the mixture cooled to 23°C.

Solution No. 2 was prepared in the following manner. To 55.0 grams of a 75% solution of (3-acrylamidopropyl) trimethyl ammonium chloride (0.20 moles) was added 21.3 grams of deionized water, 6.0 grams of PEG 200 (molecular weight 200), 0.37 ml of 30% aqueous hydrogen peroxide and 1.0 ml (5.42 x $10^{-3}$ moles) of 3-(trimethoxysilyl)propyl methacrylate. This mixture was stirred with a magnetic stir bar at about 60 rpm in a bath of water at about 23°C to provide a clear solution cooled to 23°C.

Solution No. 2 was added to Solution No. 1 while stirring with a magnetic stir bar. A thermocouple was used to monitor the temperature and observe the reaction exotherm. No polymerization exotherm was evident so the mixture was placed into a water bath and the temperature was raised from 23°C to 70°C over a time period of 35 minutes. An exotherm was evident by a rise in temperature to 73°C over a period of 1 minute and the solution became highly viscous. The reaction beaker was removed from the water bath after 50 minutes from the addition of Solution No. 2 to Solution No. 1. 152 grams of deionized water was added to reduce the polymer concentration to about 33%.

To 50 grams of the 33% polymer solution was added 2.5 ml of a 0.2% solution of hydrochloric acid. This solution was poured into two weighing dishes (about 24 grams into each dish with 80 $cm^2$ area) and the solution was dried for two days in the laboratory hood. The resultant film was very soft and flexible and slightly tacky. The absorbent capacity of the film was tested using the Centrifuge Retention Capacity test described below. The film had an absorbent capacity of 15.3 grams/gram.

Additional monomers that can be incorporated into the composition include, but are not limited to, acrylic acid or acrylamide. The composition may contain from about 15 to 99 percent by weight of a cationic quaternary amine acrylate with the balance made up of other monomers such as acrylic acid or acrylamide. The extent of neutralization of the acrylic acid can range from 5 to 95 mole percent.

In addition, the cationic absorbent binder composition may contain segments of poly(ethylene glycol) that may be grafted to the acrylate copolymer. The amount of poly(ethylene glycol) segments relative to the weight of the polymeric binder composition thereof may range from about 0.1 to about 75 weight percent ofpoly(ethylene oxide) to the weight of the polymeric binder composition. The cationic quaternary amine acrylate and poly(ethylene) glycol may also be present in the more particular amounts and ranges described previously in the specification.

Any suitable initiator can be used, with redox initiators preferred. The polymerization reaction can be done in aqueous solution, organic solvents, or miscible mixtures of organic solvents and water.

Example 8

This example is directed to a crosslinkable absorbent binder coating useful for combining with a substrate to form a humidity control product The absorbent binder coating is capable of absorbing water vapor, at 22 °C and 50% relative humidity, at a rate of at least 4 weight percent per hour. Under similar conditions particulate superabsorbent absorbs water vapor at a rate of about 0.2% per hour. The rapid rate of absorbance and the capability to form coatings with the absorbent binder coating make these materials particularly suitable for humidity control products.

The coating was prepared as follows. Two monomer solutions were prepared separately. Solution No. 1 was prepared as follows. To 55.0 grams of a 75% solution of (3-acrylamidopropyl) trimethyl ammonium chloride (0.20 moles) was added 21.3 grams of deionized water, 6.0 grams of PEG 200 (molecular weight 200). Then, 0.18 grams (1.02 x $10^{-3}$ moles) of ascorbic acid was added to the solution. This mixture was stirred with a magnetic stir bar at about 60 rpm in a bath of water at about 23 °C until the ascorbic acid was dissolved and the mixture cooled to 23 °C.

Solution No. 2 was prepared in the following manner. To 55.0 grams of a 75% solution of (3-acrylamidopropyl) trimethyl ammonium chloride (0.20 moles) was added 21.3 grams of deionized water, 6.0 grams of PEG 200 (molecular weight 200), 0.37 ml of 30% aqueous hydrogen peroxide and 1.0 ml (5.42 x 10-3 moles) of 3-(trimethoxysilyl)propyl methacrylate. This mixture was stirred with a magnetic stir bar at about 60 rpm in a bath of water at about 23 °C to provide a clear solution cooled to 23 °C.

Solution No. 2 was added to Solution No. 1 while stirring with a magnetic stir bar. A thermocouple was used to monitor the temperature and observe the reaction exotherm. No polymerization exotherm was evident so the mixture was placed into a water bath and the temperature was raised to 70 °C over a time period of 35 minutes. An exotherm was evident by a rise in temperature to 73 °C over a period of 1 minute and the solution became highly viscous. The reaction beaker was removed from the water bath after 50 minutes from the addition of Solution No.2 to Solution No. 1. 152 grams of deionized water was added to reduce the polymer concentration to about 33%.

To 50 grams of the 33% polymer solution was added 2.5 ml of a 0.2%. solution of hydrochloric acid. This solution was poured into two weighing dishes (100 $cm^2$ area) and the solution was dried for two days in the laboratory hood. The resultant film was very soft and flexible and slightly tacky. A portion of the film was cut off (0.5 gram) and soaked in 20 ml of 0.9% saline for 60 minutes. The film swelled substantially and absorbed about 12000% of the dry weight of the film.

The remainder of the film (3.806 gram) was placed in a climate-controlled room at 22 °C and 50% relative humidity. After two hours in these conditions the film was reweighed and was found to weigh 4.229 grams. The increase in weight of 0.423 grams is an increase of 11.1% in two hours or about 5.6 % per hour.

TEST METHOD FOR DETERMINING ABSORBENCY UNDER LOAD FOR COMPOSITES (AULC)

The Absorbency Under Load for Composites (AULC) is a test which measures the ability of an absorbent material to absorb a liquid (such as a 0.9 weight percent aqueous solution of sodium chloride) while under an applied load or restraining force. The AULC method provides a slight positive head of fluid for the absorbent material, which is allowed to swell under a restraining load. The material is drained under vacuum at the end of the test.

The AULC test cup is cylindrical with a height of at least 4.45 cm (1.75 inches), the inner diameter describes a cylinder, the base of which has an area of 28.19 $cm^2$ (4.37 $in^2$). The bottom of the test cup is formed by adhering a 100 mesh metal screen halving 150 micron openings to the end of the cylinder by heating the screen above the melting point of the plastic and pressing the plastic cylinder against the hot screen to melt the plastic and bond the screen to the plastic cylinder. A spacer weighing about 60 grams and having a circular diameter of about 5.99 cm (2.36 inches) is made to fit within the AULC test cup without binding. The spacer is formed with multiple cylinder holes of about 9 mm diameter, providing an open area of about 52%. A 100 mesh screen is adhered to the bottom of the spacer in a similar manner as the mesh which is attached to the bottom of the test cup or other suitable method. Weights are sized to fit on top of the spacer. The first weight should apply a load of 600 grams (in combination with the spacer), and the second weight, in combination with the first weight and the spacer disc, should apply a load of 1800 grams.

Additional equipment required includes a vacuum trap for liquid that is suctioned out of the composite material at the end of the test, shallow dishes such as Petri dishes or plastic weighing boats suitable for holding an excess amount of liquid than will be imbibed by the sample, and a thin mesh screen with a thickness between 0.3 mm and 0.75 mm and a mesh size of about 1.2 mm. The vacuum trap is adapted to apply vacuum to an area matching the dimensions of the bottom of the AULC testing cup (for example, a larger vacuum area may be selectively screened with a relatively impermeable material except in an area matching the dimensions of the bottom of the AULC cup). The vacuum applied is about 91.4kPa (27 inches of mercury)

Composite samples are cut to fit inside the AULC testing cup. Airlaid or nonwoven-based materials are cut into circles 5.97cm (2.35 inches) in diameter. Airformed samples are cut or formed into circles, each with a diameter of 5.872 cm (2.312 inches).

To carry out the test, test cup and spacer should be clean and dry. The test cup and spacer to be used in each trial should be weighed together (Measurement 1), and the mass recorded. The specimen is placed in the sample cup and the spacer is placed on top of the sample in the cup. The assembly is then weighed (Measurement 2), and the mass is recorded. The appropriate amount of weight is placed atop the spacer, if required. The spacer alone applies a force of 207Pa (0.03 pounds per square inch of area) (psia; the disc and first weight, with a net mass of 600 grams, apply a force of 2.07 kPa (0.3 psi), and the disc and both weights together, having a net mass of 1800 grams, apply a force of 6.2/kPa (0.9 psi).

The cup holding the specimen is placed in a pool of excess fluid in the shallow dish on top of the mesh screen and a one hour timer is started immediately. The level of fluid in the dish is maintained between about 1 mm and 2 mm depth. Following one hour, the specimen is removed from the fluid bath. Any fluid that may have accumulated atop the specimen should be poured off without displacing any weights atop the spacer disc. The specimen assembly is then placed on the vacuum box, with any weights still in place. Vacuum is applied to the sample for 30 seconds,

Any weights atop the spacer are then removed from the assembly and the assembly is weighed again (Measurement 3). The mass is recorded.

The dry weight of the specimen is calculated by subtracting (Measurement 1 from Measurement 2. The amount of fluid absorbed by the specimen is calculated by subtracting Measurement 2 from Measurement 3. The absorbency under load of the composite material is calculated as the amount of fluid absorbed divided by the dry weight of the

specimen.

At least three specimens of each sample should be measured, and the absorbency under load values should be averaged to obtain an overall absorbency under load for the composite sample.

TEST METHOD FOR DETERMINING STIFFNESS

A suitable technique for determining the stiffness values described herein is a Gurley Stiffness test, a description of which is set forth in TAPPI Standard Test T 543 om-94 (Bending Resistance of Paper (Gurley type tester)). A suitable testing apparatus is a Gurley Digital Stiffness Tester; Model 4171-E manufactured by Teledyne Gurley, a business having offices in Troy, New York. For purposes of the present invention, the stated Gurley stiffness values are intended to correspond to the values that would be generated by a "standard" sized sample. Accordingly, the scale readings from the Gurley stiffness tester are appropriately converted to the stiffness of a standard size sample, and are traditionally reported in terms of milligrams of force (mgf). Currently, a standard "Gurley unit" is equal to a stiffness value of 1 mgf, and may equivalently be employed to report the Gurley stiffness.

TEST METHOD FOR DETERMINING ABSORBENT CAPACITY

As used herein, the Centrifugal Retention Capacity (CRC) is a measure of the absorbent capacity of the superabsorbent material retained after being subjected to centrifugation under controlled conditions. The CRC can be measured by placing a sample of the material to be tested into a water-permeable bag which will contain the sample while allowing the test solution (0.9 percent NaCl solution) to be freely absorbed by the sample. A heat-sealable tea bag material (available from Dexter Nonwovens of Windsor Locks, Connecticut, U.S.A., as item #1234T) works well for most applications. The bag is formed by folding a 12.7 by 7.62 cm (5-inch by 3-inch) sample of the bag material in half and heat sealing two of the open edges to form a 6.35 by 7.62 cm (2.5-inch by 3-inch) rectangular pouch. The heat seals should be about 0.64cm (0.25 inch) inside the edge of the material. After the sample is placed in the pouch, the remaining open edge of the pouch is also heat-sealed. Empty bags are also made to be tested with the sample bags as controls. A sample size is chosen such that the teabag does not restrict the swelling of the material, generally with dimensions smaller than the sealed bag area (about 5.08 by 6.35cm, 2-inch by 2.5-inch). Three sample bags are tested for each material.

The sealed bags are submerged in a pan of 0.9 percent NaCl solution. After wetting, the samples remain in the solution for 60 minutes, at which time they are removed from the solution and temporarily laid on a non-absorbent flat surface.

The wet bags are then placed into the basket of a suitable centrifuge capable of subjecting the samples to a g-force of 350. (A suitable centrifuge is a Heraeus LABOFUGE 400, Heraeus Instruments, part number 75008157, available from Heraeus Infosystems GmbH, Hanau, Germany). The bags are centrifuged at a target of 1600 rpm, but within the range of 1500-1900 rpm, for 3 minutes (target g-force of 350). The bags are removed and weighed. The amount of fluid absorbed and retained by the material, taking into account the fluid retained by the bag material alone, is the Centrifugal Retention Capacity of the material, expressed as grams of fluid per gram of material.

## Claims

1. A flexible absorbent binder composition comprising a water-soluble ionic polymer capable of sufficient post-application non-radiative moisture-induced crosslinking within 10 minutes at a temperature of 120°C or less, to reach an absorbent capacity of at least one gram of fluid per gram of absorbent binder composition measured using the centrifuge retention capacity test as defined herein, the binder composition comprising:

   from 15 mass percent to 99.8 mass percent of monoethylenically unsaturated polymer units which are formed by monomers selected from the group consisting of carboxylic acid group-containing monomers, carboxylic acid salt-containing monomers, sulphonic acid group-containing monomers, sulphonic acid salt-containing monomers, phosphonic acid group-containing monomers, phosphonic acid salt-containing monomers and quaternary ammonium salt-containing monomers, and which are copolymerized with 0.1 to 20 percent by mass of acrylate or methacrylate ester units which contain an alkoxysilane functionality to form the ionic polymer; and
   0.1 to 75% by mass of polyolefin glycol and/or polyolefin oxide units which are attached to and/or blended with the copolymer of the monoethylenically unsaturated polymer units and the acrylate or methacrylate ester units which contain the alkoxysilane functionality; and
   wherein the water-soluble ionic polymer comprises at least 15 mole percent ionic polymer units.

**2.** The absorbent binder composition of Claim 1, wherein the ionic polymer has a negative charge.

**3.** The absorbent binder composition of Claim 2, wherein the ionic polymer comprises a carboxyl group-containing monomer.

**4.** The absorbent binder composition of Claim 1, wherein the ionic polymer has a positive charge.

**5.** The absorbent binder composition of Claim 1, wherein the monoethylenically unsaturated monomer comprises a carboxylic acid salt-containing monomer.

**6.** The absorbent binder composition of Claim 1, wherein the monoethylenically unsaturated monomer comprises a sulfonic acid group-containing monomer, or a quaternary ammonium salt.

**7.** The absorbent binder composition of Claim 4, wherein the ionic polymer comprises a quaternary ammonium group-containing monomer.

**8.** The absorbent binder composition of Claim 7, wherein the ionic polymer comprises a reaction product of 1) a monomer selected from the group consisting of acryloyloxyethyl-trialkyl-substituted ammonium salts, acryloyloxypropyl-trialkyl-substituted ammonium salts, acrylamidoethyl-trialkyl-substituted ammonium salts, and acrylamidopropyl-trialkyl-substitute ammonium salts, with 2) a monomer selected from the group consisting of methacryl esters which contain an alkoxysilane group and acryl esters which contain an alkoxysilane group.

**9.** The absorbent binder composition of Claim 1, wherein, upon exposure to water, the alkoxysilane functionality forms a silanol functional group which condenses to form a crosslinked polymer.

**10.** The absorbent binder composition of Claim 1, wherein the acrylate or methacrylate ester includes a trialkoxy silane functional group.

**11.** The absorbent binder composition of any of Claims 1 to 10, wherein the alkoxysilane group is a trialkoxysilane group having the following structure:

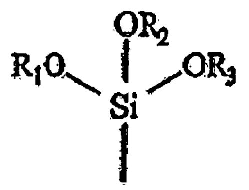

wherein $R_1$, $R_2$ and $R_3$ are alkyl groups independently having from 1 to 6 carbon atoms.

**12.** The absorbent binder composition of Claim 1, wherein the acrylate or methacrylate ester comprises at least one of methacryloxypropyl trimethoxy silane, methacryloxyethyl trimethoxy silane, methacryloxypropyl triethoxy silane, methacryloxypropyl tripropoxy silane, acryloxypropylmethyl dimethoxy silane, 3-acryloxypropyl trimethoxy silane, 3-methacryloxypropylmethyl diethoxy silane, 3-methacryloxypropylmethyl dimethoxy silane, and 3-methacryloxypropyl tris(methoxyethoxy) silane.

**13.** The absorbent binder composition of any of Claims 1-12, wherein the monoethylenically unsaturated polymer units comprise between 25% and 89.5% by weight of the absorbent binder composition.

**14.** The absorbent binder composition of Claim 13, wherein the monoethylenically unsaturated polymer units comprise between 30% and 79% by mass of the absorbent binder composition.

**15.** The absorbent binder composition of any of Claims 1-14, wherein the acrylate and/or methacrylate ester units comprise between 1.0% and 10% by mass of the absorbent binder composition.

**16.** The absorbent binder composition of Claim 1, comprising a polyolefin glycol including an olefin having 2 to 4 carbon atoms, or a polyolefm oxide including an olefin having 2 to 4 carbon atoms.

**17.** An absorbent article, comprising the absorbent binder composition of any of Claims 1-16.

**18.** A combination of an absorbent binder coating and a substrate, the coating comprising the absorbent binder composition of any of Claims 1-16.

**19.** The combination of Claim 18, further comprising a mesh layer between the absorbent binder coating and the substrate.

**20.** The combination of Claim 18 or 19, wherein the substrate comprises an article of clothing, a mirror, a camera, an electronic display, a windowsill, a window frame, a catheter, a wire, a plastic film, a nonwoven web, a carton stock, a food storage container, a bag, or a shipping container.

**21.** A personal care absorbent article comprising the combination of Claim 19 or 20, wherein the substrate includes at least one of a surge material, flap material, outer cover material, waistband material, and side panels.

**22.** The combination of Claim 18 or 19, wherein the absorbent binder coating further comprises at least one of a fragrance additive, odor-absorbing particles, an antimicrobial additive, a wound healing agent, a fungicide, a floral preservative, and a plant nutrient.

**23.** A wound dressing, a mattress pad, a medical garment, a surgical drape, a packaging material or a horticultural product comprising the combination of Claim 18 or 19.

**24.** An absorbent structure having a central region and two end regions, comprising:

a first substrate layer; and
a flexible absorbent binder bound to the substrate layer at one or more predetermined selected locations;
the flexible absorbent binder being obtainable by crosslinking the absorbent binder composition of any of Claims 1-16 on the substrate layer at the selected locations.

**25.** The absorbent structure of Claim 24, wherein the flexible absorbent binder is bound around a periphery of the substrate to form a dam.

**26.** The absorbent structure of Claim 24 or 25, wherein the flexible absorbent binder is bound to selected locations on the substrate defining channels therebetween.

**27.** An absorbent article, comprising:

a liquid-permeable fluid receiving layer;
an absorbent core; and
a liquid-impermeable outer cover;
wherein at least one of the fluid receiving layer, absorbent core and outer cover comprises the absorbent structure of any of Claims 24-26.

**28.** The absorbent article of Claim 27, wherein the absorbent core comprises the absorbent structure.

**29.** The absorbent article of Claim 27, wherein the fluid receiving layer comprises the absorbent structure.

**30.** The absorbent article of Claim 27, wherein the outer cover comprises the substrate layer of the absorbent structure and the absorbent core comprises the flexible absorbent binder formed on the substrate layer.

**31.** The absorbent article of any of Claims 27-30, further comprising superabsorbent particles combined with the flexible absorbent binder.

**32.** The absorbent article of any of Claims 27-31, further comprising a menses modifying agent combined with the flexible absorbent binder.

**33.** An absorbent article, comprising the following three layers in sequence and adjacent to each other:

a) a fluid intake layer;
b) a flexible absorbent binder layer; and
c) a support layer;

wherein the flexible absorbent binder layer is formed between the fluid intake layer and the support layer and is bound to the fluid intake layer and the support layer;
the flexible absorbent binder being formed by crosslinking the absorbent binder composition of any of Claims 1-16.

**34.** The absorbent article of Claim 33, wherein the fluid intake layer comprises one of a bodyside liner and a surge/transfer layer.

**35.** The absorbent article of Claim 33 or 34, wherein the fluid intake layer comprises a material selected from the group consisting of apertured films, spunbond webs, staple fiber webs, open-celled foams, cellulose webs and combinations thereof.

**36.** The absorbent article of Claim 35, wherein the fluid intake layer comprises a reticulated foam.

**37.** The absorbent article of any of Claims 33-36, wherein the support layer comprises one of an outer cover and a spacer.

**38.** The absorbent article of Claim 37, wherein the support layer comprises a material selected from polyolefin films, breathable polyolefin films including a polyolefin blended with a particulate filler, breathable film/nonwoven web laminates, and combinations thereof.

**39.** The absorbent article of any of Claims 27-38, wherein the absorbent article is one of a diaper, a training pant, a diaper pant, a feminine hygiene article, an adult incontinence garment, a swimwear garment, and a medical absorbent article.

**40.** A method of making an absorbent binder composition of Claim 1, comprising the steps of:

adding a first polymer selected from the group consisting of polyolefin glycols, polyolefin oxides and combinations thereof into a liquid medium;
adding a first monomer selected from the group consisting of carboxylic acid group-containing monomers, carboxylic acid salt-containing monomers, sulphonic acid group-containing monomers, sulphonic acid salt-containing monomers, phosphonic acid group-containing monomers, phosphonic acid salt-containing monomers, quaternary ammonium salt-containing monomers and combinations thereof into the liquid medium;
adding a second monomer selected from the group consisting of acrylate and methacrylate esters which contain an alkoxysilane functionality and combinations thereof into the liquid medium; and
copolymerizing the first and second monomers using the first polymer as a template, to form the absorbent binder composition.

**41.** The method of Claim 40, further comprising the steps of adding the first polymer into a first liquid medium, adding the first monomer into the first liquid medium, adding the second monomer into a second liquid medium, and combining the first liquid medium with the second liquid medium.

**42.** The method of Claim 40, further comprising the steps of adding the first polymer into a first liquid medium, adding the first monomer into a second liquid medium, adding the second monomer into the first liquid medium, and combining the first medium with the second liquid medium.

**43.** The method of Claim 40, further comprising the steps of adding the first polymer into a first liquid medium and a second liquid medium, adding the first monomer into the first liquid medium, adding the second monomer into the second liquid medium, and combining the first liquid medium with the second liquid medium.

**44.** The method of any of Claims 40-43, further comprising the steps of adding a reducing polymerization initiator to one of the first and second liquid media, and adding an oxidizing polymerization initiator to the other of the first and second liquid media.

**45.** The method of any of Claims 40-44, further comprising the step of crosslinking the absorbent binder composition in the absence of radiation, at a temperature of about 120°C or less.

**46.** A method of making an absorbent article of any of Claims 33-39, comprising the steps of:

providing a fluid intake layer;
providing a support layer;
applying an absorbent binder composition to at least one of the fluid intake layer and the support layer;
bringing the layers together so that the absorbent binder composition contacts both the fluid intake layer and the support layer; and
crosslinking the absorbent binder composition to provide the flexible absorbent binder layer bound to both the fluid intake layer and the support layer.

**47.** The method of Claim 46, wherein the crosslinking is performed without radiation at a temperature of 120°C or less.

**Patentansprüche**

**1.** Flexible absorptionsfähige Bindemittelzusammensetzung, welche ein wasserlösliches ionisches Polymer umfasst, welches zu einer ausreichenden Nachanwendungs- und feuchtigkeitsinduzierten Vernetzung ohne Strahlung innerhalb von 10 Minuten bei einer Temperatur von 120 °C oder weniger in der Lage ist, um eine Absorptionskapazität von mindestens ein Gramm Fluid pro Gramm absorptionsfähiger Bindemittelzusammensetzung zu erreichen, gemessen mittels des Zentrifugationsrückhaltekapazitätstests wie hierin definiert, wobei die Bindemittelzusammensetzung umfasst:

von 15 Massenprozent bis 99,8 Massenprozent von monoethylenisch ungesättigten Polymereinheiten, welche aus Monomeren gebildet sind, die ausgewählt sind aus der Gruppe bestehend aus Carboxylsäuregruppen enthaltenden Monomeren, Carboxylsäuresalzhaltigen Monomeren, Sulphonsäuregruppen enthaltenden Monomeren, Sulphonsäuresalzhaltigen Monomeren, Phosphonsäuregruppen enthaltenden Monomeren, Phosphonsäuresalzhaltigen Monomeren und quaternäres Ammoniumsalzhaltigen Monomeren, und welche mit 0,1 bis 20 Massenprozent mit Acrylat- oder Methacrylatestereinheiten copolymerisiert sind, welche eine Alkoxysilan-Funktionalität beinhalten, um das ionische Polymer zu bilden; und
0,1 bis 75 Massenprozent Polyolefinglycol und/oder Polyolefinoxideinheiten, welche an dem Copolymer der monoethylenisch ungesättigten Polymereinheiten und den Acrylat- oder Methacrylatestereinheiten, welche die Alkoxysilan-Funktionalität enthalten, befestigt sind und/oder gemischt sind; und
wobei das wasserlösliche ionische Polymer mindestens 15 Molprozent ionische Polymereinheiten umfasst.

**2.** Absorptionsfähige Bindemittelzusammensetzung gemäß Anspruch 1, wobei das ionische Polymer eine negative Ladung aufweist.

**3.** Absorptionsfähige Bindemittelzusammensetzung gemäß Anspruch 2, wobei das ionische Polymer ein Carboxylgruppen enthaltendes Monomer umfasst.

**4.** Absorptionsfähige Bindemittelzusammensetzung gemäß Anspruch 1, wobei das ionische Polymer eine positive Ladung aufweist.

**5.** Absorptionsfähige Bindemittelzusammensetzung gemäß Anspruch 1, wobei das monoethylenisch ungesättigte Monomer ein Carboxylsäuresalz enthaltendes Monomer umfasst.

**6.** Absorptionsfähige Bindemittelzusammensetzung gemäß Anspruch 1, wobei das monoethylenisch ungesättigte Monomer ein Sulfonsäuregruppen enthaltendes Monomer oder ein quaternäres Ammoniumsalz umfasst.

**7.** Absorptionsfähige Bindemittelzusammensetzung gemäß Anspruch 4, wobei das ionische Polymer ein quaternäre Ammoniumgruppen enthaltendes Monomer umfasst.

**8.** Absorptionsfähige Bindemittelzusammensetzung gemäß Anspruch 7, wobei das ionische Polymer ein Reaktionsprodukt umfasst aus 1) einem Monomer, welches ausgewählt ist aus der Gruppe bestehend aus Acryloyloxyethyl-Trialkyl-substituierten Ammoniumsalzen, Acryloyloxypropyl-Trialkyl-substitiuierten Ammoniumsalzen, Acrylamidoethyl-Trialkyl-substituierten Ammoniumsalzen und Acrylamidopropyl-Trialkyl-substituierten Ammoniumsalzen mit 2) einem Monomer, welches ausgewählt ist aus der Gruppe bestehend aus Methacrylestern, welche eine Alkoxysilangruppe enthalten, und Acrylestern, welche eine Alkoxysilangruppe enthalten.

9. Absorptionsfähige Bindemittelzusammensetzung gemäß Anspruch 1, wobei, wenn sie Wasser ausgesetzt wird, die Alkoxysilan-Funktionalität eine Silanolfunktionale Gruppe bildet, welche kondensiert, um ein vernetztes Polymer zu bilden.

10. Absorptionsfähige Bindemittelzusammensetzung gemäß Anspruch 1, wobei der Acrylatester oder der Methacrylatester eine Trialkoxy-Silan-funktionale Gruppe beinhaltet.

11. Absorptionsfähige Bindemittelzusammensetzung gemäß einem der Ansprüche 1 bis 10, wobei die Alkoxysilangruppe eine Trialkoxysilangruppe ist, welche die folgende Struktur aufweist:

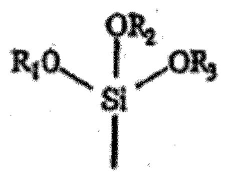

wobei $R_1$, $R_2$ und $R_3$ Alkylgruppen sind, welche unabhängig von 1 bis 6 Kohlenstoffatome aufweisen.

12. Absorptionsfähige Bindemittelzusammensetzung gemäß Anspruch 1, wobei der Acrylatester oder der Methacrylatester mindestens eines umfasst aus Methacryloxypropyl-Trimethoxy-Silan, Methacryloxyethyl-Trimethoxy-Silan, Methacryloxypropyl-Triethoxy-Silan, Methacryloxypropyl-Tripropoxy-Silan, Acryloxypropylmethyl-Dimethoxy-Silan, 3-Acryloxypropyl-Trimethoxy-Silan, 3-Methacryloxypropylmethyl-Diethoxy-Silan, 3-Methacryloxypropylmethyl-Dimethoxy-Silan und 3-Methacryloxypropyl-Tris(Methoxyethoxy)-Silan.

13. Absorptionsfähige Bindemittelzusammensetzung gemäß einem der Ansprüche 1-12, wobei die monoethylenisch ungesättigten Polymereinheiten zwischen 25 und 89,5 Gew.-% der absorptionsfähigen Bindemittelzusammensetzung umfassen.

14. Absorptionsfähige Bindemittelzusammensetzung gemäß Anspruch 13, wobei die monoethylenisch ungesättigten Polymereinheiten zwischen 30 und 79 Massen-% der absorptionsfähigen Bindemittelzusammensetzung umfassen.

15. Absorptionsfähige Bindemittelzusammensetzung gemäß einem der Ansprüche 1-14, wobei die Acrylat- und/oder Methacrylatestereinheiten zwischen 1,0 und 10 Massen-% der absorptionsfähigen Bindemittelzusammensetzung umfassen.

16. Absorptionsfähige Bindemittelzusammensetzung gemäß Anspruch 1, welche ein Polyolefinglycol umfasst, welches ein Olefin mit 2 bis 4 Kohlenstoffatomen beinhaltet, oder ein Polyolefinoxid umfasst, welches ein Olefin mit 2 bis 4 Kohlenstoffatomen beinhaltet.

17. Absorptionsfähiger Artikel, welcher die absorptionsfähige Bindemittelzusammensetzung gemäß einem der Ansprüche 1 bis 16 umfasst.

18. Kombination einer absorptionsfähigen Bindemittelbeschichtung und eines Substrats, wobei die Beschichtung die absorptionsfähige Bindemittelzusammensetzung gemäß einem der Ansprüche 1 bis 16 umfasst.

19. Kombination gemäß Anspruch 18, welche des Weiteren eine Maschenschicht zwischen der absorptionsfähigen Bindemittelbeschichtung und dem Substrat umfasst.

20. Kombination gemäß Anspruch 18 oder 19, wobei das Substrat einen Artikel einer Bekleidung, eines Spiegels, einer Kamera, eine elektronischen Displays, einer Fensterbank, eines Fensterrahmens, eines Katheters, eines Drahts, eines Plastikfilms, einer Vliesbahn, eines Kartonmaterials, eines Nahrungsvorratsbehälters, einer Tasche oder eines Versandbehälters umfasst.

21. Körperpflegeartikel, welcher die Kombination gemäß Anspruch 19 oder 20 umfasst, wobei das Substrat mindestens eines beinhaltet von einem Durchflussmaterial, einem Laschenmaterial, einer äußeren Deckschicht, einem Taillenbundmaterial und Seitenbahnen.

**22.** Kombination gemäß Anspruch 18 oder 19, wobei die absorptionsfähige Bindemittelbeschichtung des Weiteren mindestens eines aus einem Duftzusatz, Geruch absorbierenden Partikeln, einem antimikrobiellen Zusatz, ein Wundheilungsmittel, einem Fungizid, einen Blumenkonservierungsstoff und einem Pflanzennährstoff umfasst.

**23.** Wundauflage, Matratzenauflage, medizinische Bekleidung, ein chirurgisches Tuch, ein Verpackungsmaterial oder ein Gartenprodukt, welches die Kombination gemäß Anspruch 18 oder 19 umfasst.

**24.** Absorptionsfähige Struktur, welche einen zentralen Bereich und zwei Endbereiche umfasst, welche umfasst:

eine erste Substratschicht; und
ein flexibles absorptionsfähiges Bindemittel, welches an die Substratschicht an einer oder mehreren vorbestimmten ausgewählten Stellen gebunden ist;
wobei das flexible absorptionsfähige Bindemittel erhältlich ist durch Vernetzen der absorptionsfähigen Bindemittelzusammensetzung gemäß einem der Ansprüche 1 bis 16 auf der Substratschicht an den ausgewählten Stellen.

**25.** Absorptionsfähige Struktur gemäß Anspruch 24, wobei das flexible absorptionsfähige Bindemittel um eine Peripherie des Substrats gebunden ist, um einen Damm zu bilden.

**26.** Absorptionsfähige Struktur gemäß Anspruch 24 oder 25, wobei das flexible absorptionsfähige Bindemittel an ausgewählten Stellen auf dem Substrat gebunden ist, wobei dazwischen Kanäle definiert sind.

**27.** Absorptionsfähiger Artikel, welcher umfasst:

eine flüssigkeitsdurchlässige Fluid aufnehmende Schicht;
einen absorptionsfähigen Kern; und
eine flüssigkeitsundurchlässige äußere Deckschicht;
wobei mindestens eines aus der Fluid aufnehmenden Schicht, dem absorptionsfähigen Kern und der äußeren Deckschicht die absorptionsfähige Struktur gemäß einem der Ansprüche 24 bis 26 umfasst.

**28.** Absorptionsfähiger Artikel gemäß Anspruch 27, wobei der absorptionsfähige Kern die absorptionsfähige Struktur umfasst.

**29.** Absorptionsfähiger Artikel gemäß Anspruch 27, wobei die Fluid aufnehmende Schicht die absorptionsfähige Struktur umfasst.

**30.** Absorptionsfähiger Artikel gemäß Anspruch 27, wobei die äußere Deckschicht die Substratschicht der absorptionsfähigen Struktur umfasst, und wobei der absorptionsfähige Kern das flexible absorptionsfähige Bindemittel umfasst, das auf der Substratschicht gebildet ist.

**31.** Absorptionsfähiger Artikel gemäß einem der Ansprüche 27 bis 30, welcher des Weiteren superabsorptionsfähige Partikel kombiniert mit dem flexiblen absorptionsfähigen Bindemittel umfasst.

**32.** Absorptionsfähiger Artikel gemäß einem der Ansprüche 27 bis 31, welcher des Weiteren ein Menses modifizierendes Mittel kombiniert mit dem flexiblen absorptionsfähigen Bindemittel umfasst.

**33.** Absorptionsfähiger Artikel, welcher die folgenden drei Schichten in Reihenfolge und angrenzend aneinander umfasst:

a) eine Fluideinnahmeschicht;
b) eine Schicht aus flexiblem absorptionsfähigen Bindemittel; und
c) eine Trageschicht;

wobei die Schicht aus flexiblem absorptionsfähigen Bindemittel zwischen der Fluideinnahmeschicht und der Trageschicht gebildet ist, und an die Fluideinnahmeschicht und die Trageschicht gebunden ist;
wobei das flexible absorptionsfähige Bindemittel durch Vernetzen der absorptionsfähigen Bindemittelzusammensetzung gemäß einem der Ansprüche 1 bis 16 gebildet ist.

**34.** Absorptionsfähiger Artikel gemäß Anspruch 33, wobei die Fluideinnahmeschicht eines von einer körperseitigen

Auskleidung und einer Durchfluss-/Transferschicht umfasst.

**35.** Absorptionsfähiger Artikel gemäß Anspruch 33 oder 34, wobei die Fluideinnahmeschicht ein Material umfasst, welches ausgewählt ist aus der Gruppe bestehend aus perforierten Filmen, Spunbond-Bahnen, Stapelfaser-Bahnen, offenzelligen Schäumen, Zellulose-Bahnen und Kombinationen davon.

**36.** Absorptionsfähiger Artikel gemäß Anspruch 35, wobei die Fluideinnahmeschicht einen netzartigen Schaum umfasst.

**37.** Absorptionsfähiger Artikel gemäß einem der Ansprüche 33 bis 36, wobei die Trageschicht eines von einer äußeren Deckschicht und einem Abstandshalter umfasst.

**38.** Absorptionsfähiger Artikel gemäß Anspruch 37, wobei die Trageschicht ein Material umfasst, welches ausgewählt ist aus Polyolefin-Filmen, atmungsaktiven Polyolefin-Filmen, welche ein Polyolefin beinhalten, welches mit einem partikulären Füllmittel gemischt ist, atmungsaktiven Film/Vliesbahnlaminaten und Kombinationen davon.

**39.** Absorptionsfähiger Artikel gemäß einem der Ansprüche 27 bis 38, wobei der absorptionsfähige Artikel eines ist von einer Windel, einem Trainingshöschen, einer Windelhose, einem Damenhygieneartikel, einer Erwachseneninkontinenzbekleidung, einer Schwimmbekleidung und einem absorptionsfähigen Medizinartikel.

**40.** Verfahren zur Herstellung einer absorptionsfähigen Bindemittelzusammensetzung gemäß Anspruch 1, welches die Schritte umfasst:

Hinzugeben eines ersten Polymer, welches ausgewählt ist aus der Gruppe bestehend aus Polyolefinglycolen, Polyolefinoxiden und Kombinationen davon, in ein flüssiges Medium;
Hinzugeben eines ersten Monomers, welches ausgewählt ist aus der Gruppe bestehend aus Carboxylsäuregruppen enthaltenden Monomeren, Carboxylsäuresalzhaltigen Monomeren, Sulphonsäuregruppen enthaltenden Monomeren, Sulphonsäuresalzhaltigen Monomeren, Phosphonsäuregruppen enthaltenden Monomeren, Phosphonsäuresalzhaltigen Monomeren, quaternäres Ammoniumsalzhaltigen Monomeren und Kombinationen davon, in das flüssige Medium;
Hinzugeben eines zweiten Monomers, welches ausgewählt ist aus der Gruppe bestehend aus Acrylat- und Methacrylatestern, welche eine Alkoxysilan-Funktionalität aufweisen, und Kombinationen davon, in das flüssige Medium; und
Copolymerisieren des ersten und zweiten Monomers unter Verwendung des ersten Polymers als eine Vorlage, um die absorptionsfähige Bindemittelzusammensetzung zu bilden.

**41.** Verfahren gemäß Anspruch 40, welches des Weiteren die Schritte des Hinzufügens des ersten Polymers in ein erstes flüssiges Medium, Hinzufügen des ersten Monomers in das erste flüssige Medium, Hinzufügen des zweiten Monomers in ein zweites flüssiges Medium und Kombinieren des ersten flüssigen Mediums mit dem zweiten flüssigen Medium umfasst.

**42.** Verfahren gemäß Anspruch 40, welches des Weiteren die Schritte des Hinzufügens des ersten Polymers in ein erstes flüssiges Medium, Hinzufügen des ersten Monomers in ein zweites flüssiges Medium, Hinzufügen des zweiten Monomers in das erste flüssige Medium, und Kombinieren des ersten Mediums mit dem zweiten flüssigen Medium umfasst.

**43.** Verfahren gemäß Anspruch 40, welches des Weiteren die Schritte des Hinzufügens des ersten Polymers in ein erstes flüssiges Medium und ein zweites flüssiges Medium, Hinzufügen des ersten Monomers in das erste flüssige Medium, Hinzufügen des zweiten Monomers in das zweite flüssige Medium und Kombinieren des ersten flüssigen Mediums mit dem zweiten flüssigen Medium umfasst.

**44.** Verfahren gemäß einem der Ansprüche 40-43, welches des Weiteren die Schritte des Hinzufügens eines reduzierenden Polymerisationsinitiators zu einem des ersten und zweiten flüssigen Mediums, und Hinzufügen eines oxidierenden Polymerisationsinitiators zu dem anderen des ersten und zweiten flüssigen Mediums.

**45.** Verfahren gemäß einem der Ansprüche 40-44, welches des Weiteren den Schritt des Vernetzens der absorptionsfähigen Bindemittelzusammensetzung in Abwesenheit von Strahlung umfasst, bei einer Temperatur von ungefähr 120 °C oder weniger.

**46.** Verfahren zur Herstellung eines absorptionsfähigen Artikels gemäß einem der Ansprüche 33-39, welches die Schritte umfasst:

Bereitstellen einer Fluideinnahmeschicht;
Bereitstellen einer Trageschicht;
Aufbringen einer absorptionsfähigen Bindemittelzusammensetzung auf mindestens eine der Fluideinnahmeschicht und der Trageschicht;
Zusammenbringen der Schichten, so dass die absorptionsfähige Bindemittelzusammensetzung sowohl die Fluideinnahmeschicht als auch die Trageschicht kontaktiert; und
Vernetzen der absorptionsfähigen Bindemittelzusammensetzung, um die flexible absorptionsfähige Bindemittelschicht bereitzustellen, die sowohl an die Fluideinnahmeschicht als auch an die Trageschicht gebunden ist.

**47.** Verfahren gemäß Anspruch 46, wobei das Vernetzen ohne Strahlung bei einer Temperatur von 120 °C oder weniger durchgeführt wird.

## Revendications

**1.** Composition de liant absorbant souple comprenant un polymère ionique hydrosoluble susceptible d'une réticulation suffisante induite par humidité non radiative post-application en 10 minutes à une température de 120 °C ou moins, pour atteindre une capacité d'absorption d'au moins un gramme de fluide par gramme de composition de liant absorbant mesurée en utilisant le test de capacité de rétention centrifuge tel que défini dans ce document, la composition de liant comprenant :

de 15 pour cent en masse à 99,8 pour cent en masse d'unités de polymère non saturé en ce qui concerne le monoéthylène qui sont formées par des monomères sélectionnés à partir du groupe constitué par des monomères contenant un groupe acide carboxylique, des monomères contenant un sel d'acide carboxylique, des monomères contenant un groupe acide sulfonique, des monomères contenant un sel d'acide sulfonique, des monomères contenant un groupe acide phosphonique, des monomères contenant un sel d'acide phosphonique et des monomères contenant un sel d'ammonium quaternaire, et qui sont copolymérisés avec 0,1 à 20 pour cent en masse d'unités d'ester d'acrylate ou de méthacrylate qui contiennent une fonctionnalité alcoxysilane pour former le polymère ionique ; et
0,1 à 75 % en masse d'unités d'oxyde de polyoléfine et/ou de glycol de polyoléfine qui sont attachées à et/ou mélangées au copolymère des unités de polymère non saturé en ce qui concerne le monoéthylène et des unités d'ester d'acrylate ou de méthacrylate qui contiennent la fonctionnalité alcoxysilane ; et
dans laquelle le polymère ionique hydrosoluble comprend au moins 15 pour cent en mole d'unités de polymère ionique.

**2.** Composition de liant absorbant selon la revendication 1, dans laquelle le polymère ionique a une charge négative.

**3.** Composition de liant absorbant selon la revendication 2, dans laquelle le polymère ionique comprend un monomère contenant un groupe carboxyle.

**4.** Composition de liant absorbant selon la revendication 1, dans laquelle le polymère ionique a une charge positive.

**5.** Composition de liant absorbant selon la revendication 1, dans laquelle le monomère non saturé en ce qui concerne le monoéthylène comprend un monomère contenant un sel d'acide carboxylique.

**6.** Composition de liant absorbant selon la revendication 1, dans laquelle le monomère non saturé en ce qui concerne le monoéthylène comprend un monomère contenant un groupe acide sulfonique, ou un sel d'ammonium quaternaire.

**7.** Composition de liant absorbant selon la revendication 4, dans lequel le polymère ionique comprend un monomère contenant un groupe ammonium quaternaire.

**8.** Composition de liant absorbant selon la revendication 7, dans lequel le polymère ionique comprend un produit de réaction de 1) un monomère sélectionné à partir du groupe constitué par des sels d'ammonium à acryloyloxyéthyltrialkyle substitué, des sels d'ammonium à acryloyloxypropyl-trialkyle substitué, des sels d'ammonium à acrylamidoéthyltrialkyle substitué, et des sels d'ammonium à acrylamidopropyl-trialkyle substitué, avec 2) un monomère

sélectionné à partir du groupe constitué par des esters de méthacryle qui contiennent un groupe alcoxysilane et des esters d'acryle qui contiennent un groupe alcoxysilane.

9. Composition de liant absorbant selon la revendication 1, dans laquelle, lors d'une exposition à de l'eau, la fonctionnalité alcoxysilane forme un groupe fonctionnel silanol qui se condense pour former un polymère réticulé.

10. Composition de liant absorbant selon la revendication 1, dans laquelle l'ester d'acrylate ou de méthacrylate inclut un groupe fonctionnel trialcoxy silane.

11. Composition de liant absorbant selon l'une quelconque des revendications 1 à 10, dans laquelle le groupe alcoxysilane est un groupe trialcoxysilane ayant la structure suivante :

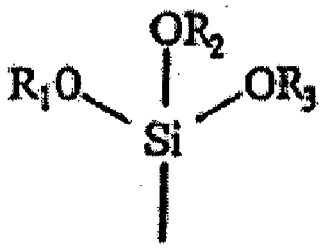

dans laquelle $R_1$, $R_2$ et $R_3$ sont des groupes alkyle ayant indépendamment de 1 à 6 atomes de carbone.

12. Composition de liant absorbant selon la revendication 1, dans laquelle l'ester d'acrylate ou de méthacrylate comprend au moins l'un de méthacryloxypropyle triméthoxysilane, méthacryloxyéthyle triméthoxysilane, méthacryloxypropyle triéthoxysilane, méthacryloxypropyle tripropoxy silane, acryloxypropylméthyle diméthoxysilane, 3-acryloxypropyle triméthoxysilane, 3-méthacryloxypropylméthyle diéthoxysilane, 3-méthacryloxypropylméthyle diméthoxysilane, et 3-méthacryloxypropyle tris(méthoxyéthoxy) silane.

13. Composition de liant absorbant selon l'une quelconque des revendications 1 à 12, dans laquelle les unités de polymère non saturé en ce qui concerne le monoéthylène comprennent entre 25 % et 89,5 % en poids de la composition de liant absorbant.

14. Composition de liant absorbant selon la revendication 13, dans laquelle les unités de polymère non saturé en ce qui concerne le monoéthylène comprennent entre 30 % et 79 % en masse de la composition de liant absorbant.

15. Composition de liant absorbant selon l'une quelconque des revendications 1 à 14, dans laquelle les unités d'ester d'acrylate et/ou de méthacrylate comprennent entre 1,0 % et 10 % en masse de la composition de liant absorbant.

16. Composition de liant absorbant selon la revendication 1, comprenant un glycol de polyoléfine incluant une oléfine ayant 2 à 4 atomes de carbone, ou un oxyde de polyoléfine incluant une oléfine ayant de 2 à 4 atomes de carbone.

17. Article absorbant, comprenant la composition de liant absorbant selon l'une quelconque des revendications 1 à 16.

18. Combinaison d'un revêtement de liant absorbant et d'un substrat, le revêtement comprenant la composition de liant absorbant selon l'une quelconque des revendications 1 à 16.

19. Combinaison selon la revendication 18, comprenant en outre une couche de mailles entre le revêtement de liant absorbant et le substrat.

20. Combinaison selon la revendication 18 ou 19, dans laquelle le substrat comprend un article vestimentaire, un miroir, un appareil de prise de vues, un afficheur électronique, un appui de fenêtre, un châssis de fenêtre, un cathéter, un câble, un film de plastique, un tissu non tissé, une matière cartonnée, un conteneur de stockage de nourriture, un sac, ou un conteneur de chargement.

21. Article absorbant pour hygiène personnelle comprenant la combinaison de la revendication 19 ou 20, dans lequel le substrat inclut au moins une d'une matière anti-fuite, d'une matière à rabat, d'une matière de couverture extérieure,

d'une matière de ceinture, et de panneaux latéraux.

**22.** Combinaison selon la revendication 18 ou 19, dans laquelle le revêtement de liant absorbant comprend en outre au moins l'un d'un additif de parfum, de particules absorbant les odeurs, d'un additif antimicrobien, d'un agent de cicatrisation, d'un fongicide, d'un conservateur floral, et d'une substance nutritive pour plante.

**23.** Pansement, coussin de matelas, vêtement médical, champ chirurgical, matière d'emballage ou produit horticole comprenant la combinaison selon la revendication 18 ou 19.

**24.** Structure absorbante ayant une région centrale et deux régions d'extrémité, comprenant :

une première couche de substrat ; et
un liant absorbant souple attaché à la couche de substrat au niveau d'un ou plusieurs emplacements sélectionnés prédéterminés ;
le liant absorbant souple pouvant être obtenu en réticulant la composition de liant absorbant selon l'une quelconque des revendications 1 à 16 sur la couche de substrat au niveau des emplacements sélectionnés.

**25.** Structure absorbante selon la revendication 24, dans laquelle le liant absorbant souple est lié autour d'une périphérie du substrat pour former un barrage.

**26.** Structure absorbante selon la revendication 24 ou 25, dans laquelle le liant absorbant souple est lié à des emplacements sélectionnés sur le substrat définissant des canaux entre ces derniers.

**27.** Article absorbant, comprenant :

une couche de réception de fluide perméable au liquide ;
une âme absorbante ; et
une couverture extérieure imperméable au liquide ;
dans lequel au moins une de la couche de réception de fluide, de l'âme absorbante et de la couverture extérieure comprend la structure absorbante selon l'une quelconque des revendications 24 à 26.

**28.** Article absorbant selon la revendication 27, dans lequel l'âme absorbante comprend la structure absorbante.

**29.** Article absorbant selon la revendication 27, dans lequel la couche de réception de fluide comprend la structure absorbante.

**30.** Article absorbant selon la revendication 27, dans lequel la couverture extérieure comprend la couche de substrat de la structure absorbante et l'âme absorbante comprend le liant absorbant souple formé sur la couche de substrat.

**31.** Article absorbant selon l'une quelconque des revendications 27 à 30, comprenant en outre des particules superabsorbantes combinées au liant absorbant souple.

**32.** Article absorbant selon l'une quelconque des revendications 27 à 31, comprenant en outre un agent de modification des menstrues combiné au liant absorbant souple.

**33.** Article absorbant, comprenant les trois couches suivantes en séquence et adjacentes les unes aux autres :

a) une couche d'entrée de fluide ;
b) une couche de liant absorbant souple ; et
c) une couche de support ;

dans lequel la couche de liant absorbant souple est formée entre la couche d'entrée de fluide et la couche de support, et est liée à la couche d'entrée de fluide et à la couche de support ;
le liant absorbant souple étant formé en réticulant la composition de liant absorbant selon l'une quelconque des revendications 1 à 16.

**34.** Article absorbant selon la revendication 33, dans lequel la couche d'entrée de fluide comprend l'une d'une doublure côté corps et d'une couche anti-fuite / de transfert.

**35.** Article absorbant selon la revendication 33 ou 34, dans lequel la couche d'entrée de fluide comprend une matière sélectionnée à partir du groupe constitué par des films à ouvertures, des tissus de filé-lié, des tissus à fibres courtes, des mousses à alvéoles ouverts, des tissus de cellulose et des combinaisons de ceux-ci.

**36.** Article absorbant selon la revendication 35, dans lequel la couche d'entrée de fluide comprend une mousse réticulée.

**37.** Article absorbant selon l'une quelconque des revendications 33 à 36, dans lequel la couche de support comprend l'une d'une couverture extérieure et d'une pièce d'écartement.

**38.** Article absorbant selon la revendication 37, dans lequel la couche de support comprend une matière sélectionnée à partir de films de polyoléfine, de films de polyoléfine respirables incluant une polyoléfine mélangée avec un agent de remplissage particulaire, des stratifiés de tissu de film respirable / non tissé, et des combinaisons de ceux-ci.

**39.** Article absorbant selon l'une quelconque des revendications 27 à 38, dans laquelle l'article absorbant est l'un d'une couche, d'une couche-culotte, d'un article d'hygiène féminine, d'une protection adulte contre l'incontinence, d'un vêtement de bain, et d'un article absorbant médical.

**40.** Procédé de fabrication d'une composition de liant absorbant selon la revendication 1, comprenant les étapes de :

ajout d'un premier polymère sélectionné à partir du groupe constitué de glycols de polyoléfine, d'oxydes de polyoléfine et de combinaisons de ceux-ci dans un milieu liquide ;
ajout d'un premier monomère sélectionné à partir du groupe constitué de monomères contenant un groupe acide carboxylique, de monomères contenant un sel d'acide carboxylique, de monomères contenant un groupe acide sulfonique, de monomères contenant un sel d'acide sulfonique, de monomères contenant un groupe acide phosphonique, de monomères contenant un sel d'acide phosphonique, de monomères contenant un sel d'ammonium quaternaire et de combinaisons de ceux-ci dans le milieu liquide ;
ajout d'un second monomère sélectionné à partir du groupe constitué par des esters d'acrylate et de méthacrylate qui contiennent une fonctionnalité alcoxysilane et des combinaisons de ceux-ci dans le milieu liquide ; et
copolymérisation des premier et second monomères en utilisant le premier polymère en tant que modèle, pour former la composition de liant absorbant.

**41.** Procédé selon la revendication 40, comprenant en outre les étapes d'ajout du premier polymère dans un premier milieu liquide, d'ajout du premier monomère dans le premier milieu liquide, d'ajout du second monomère dans un second milieu liquide, et de combinaison du premier milieu liquide avec le second milieu liquide.

**42.** Procédé selon la revendication 40, comprenant en outre les étapes d'ajout du premier polymère dans un premier milieu liquide, d'ajout du premier monomère dans un second milieu liquide, d'ajout du second monomère dans le premier milieu liquide, et combinaison du premier milieu avec le second milieu liquide.

**43.** Procédé selon la revendication 40, comprenant en outre les étapes d'ajout du premier polymère dans un premier milieu liquide et un second milieu liquide, d'ajout du premier monomère dans le premier milieu liquide, d'ajout du second monomère dans le second milieu liquide, et combinaison du premier milieu liquide avec le second milieu liquide.

**44.** Procédé selon l'une quelconque des revendications 40 à 43, comprenant en outre les étapes d'ajout d'un initiateur de polymérisation par réduction à un des premier et second milieux liquides, et d'ajout d'un initiateur de polymérisation par oxydation à l'autre des premier et second milieux liquides.

**45.** Procédé selon l'une quelconque des revendications 40 à 44, comprenant en outre l'étape de réticulation de la composition de liant absorbant en l'absence de rayonnement, à une température d'environ 120 °C ou moins.

**46.** Procédé de fabrication d'un article absorbant selon l'une quelconque des revendications 33 à 39, comprenant les étapes de :

fourniture d'une couche d'entrée de fluide ;
fourniture d'une couche de support ;
application d'une composition de liant absorbant à au moins une de la couche d'entrée de fluide et de la couche de support ;

rassemblement des couches de sorte que la composition de liant absorbant entre en contact tant avec la couche d'entrée de fluide que la couche de support ; et
réticulation de la composition de liant absorbant pour fournir la couche de liant absorbant souple liée tant à la couche d'entrée de fluide qu'à la couche de support.

**47.** Procédé selon la revendication 46, dans lequel la réticulation est effectuée sans rayonnement à une température de 120 °C ou moins.

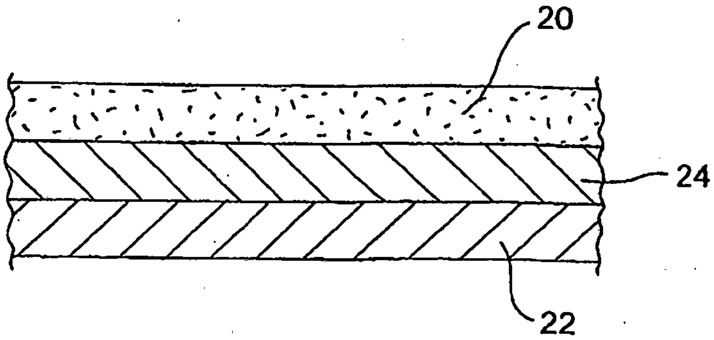


FIG. 1

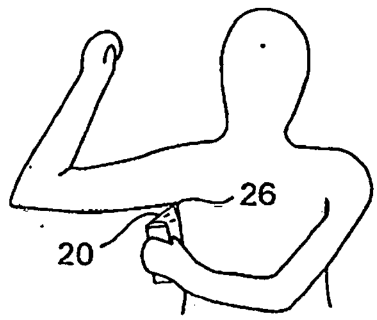


FIG. 2

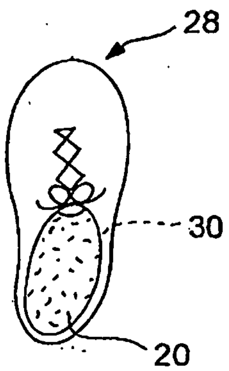


FIG. 3

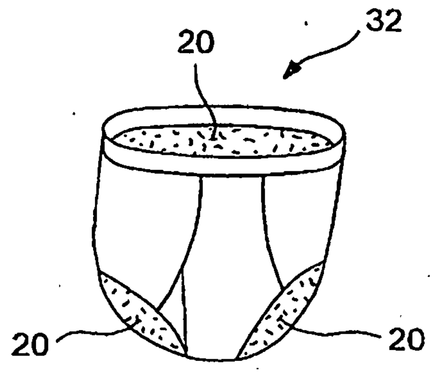


FIG. 4

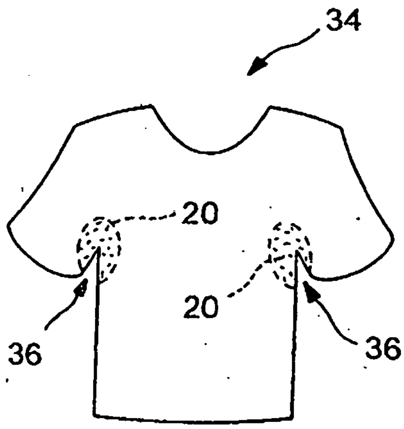


FIG. 5

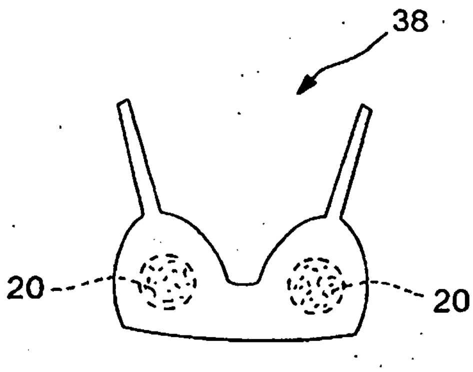


FIG. 6

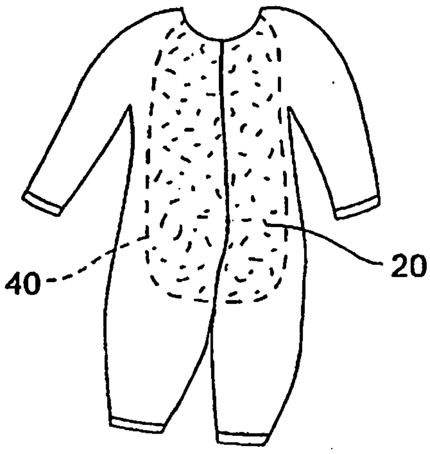


FIG. 7

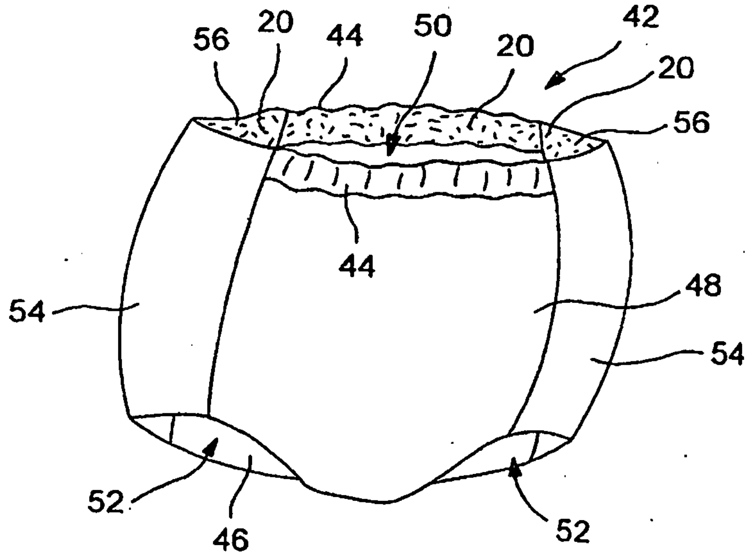


FIG. 8

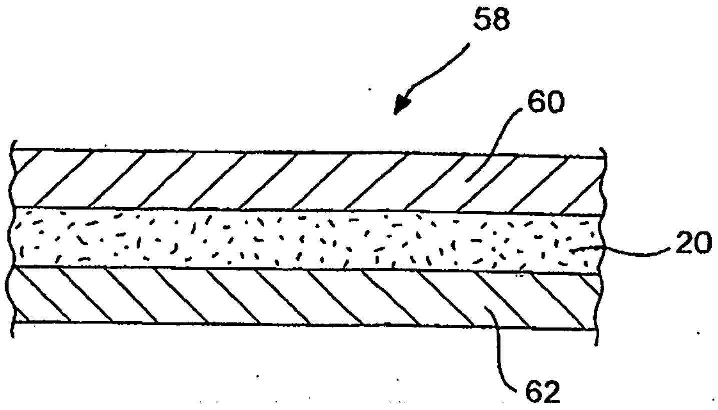


FIG. 9

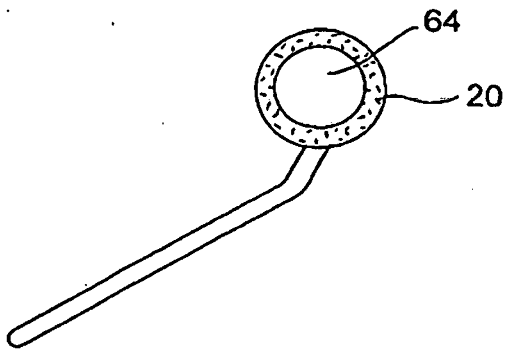


FIG. 10

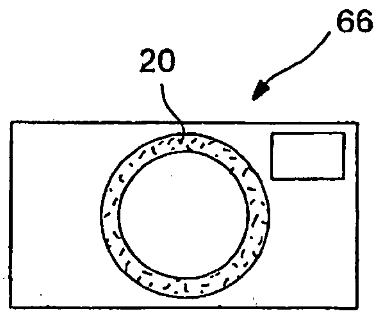


FIG. 11

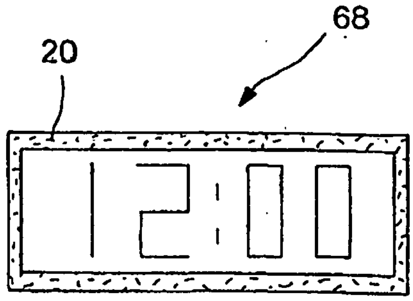


FIG. 12

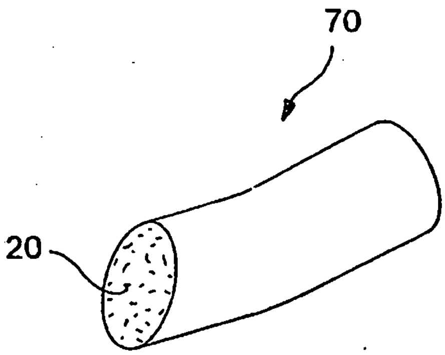


FIG. 13

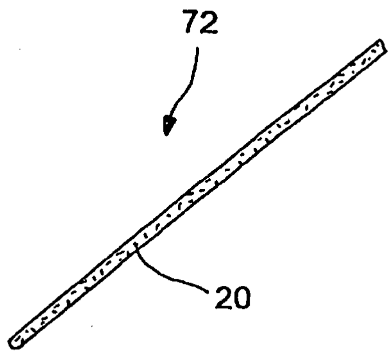


FIG. 14

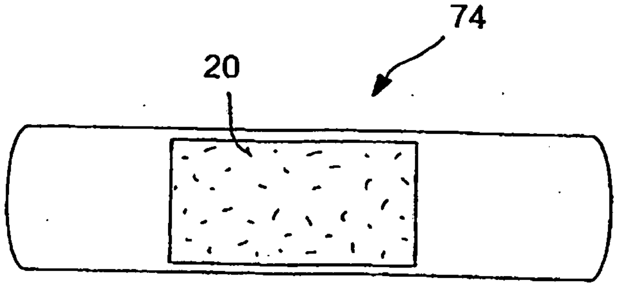


FIG. 15

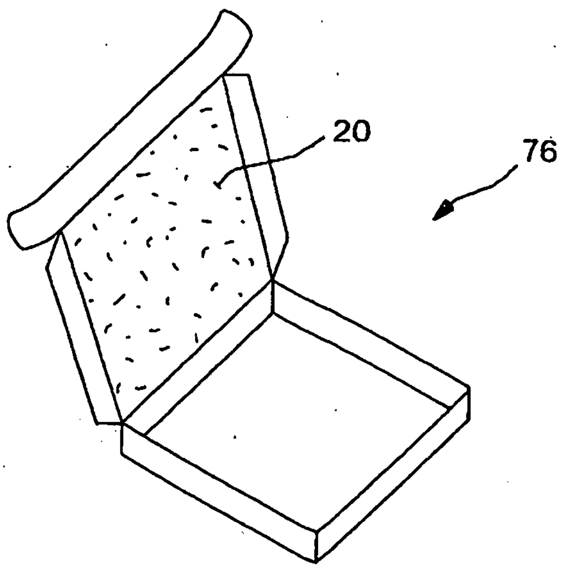


FIG. 16

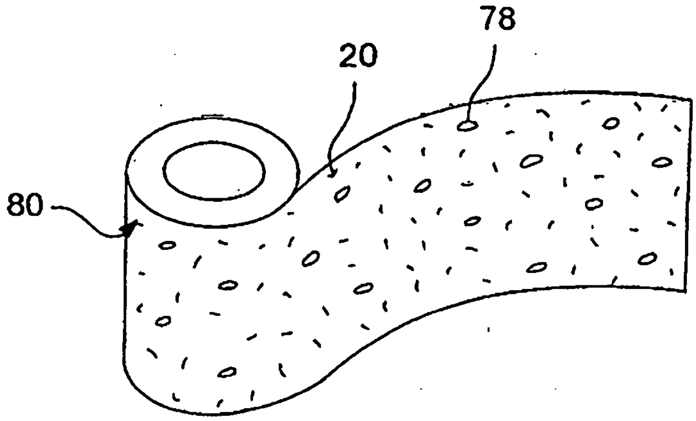


FIG. 17

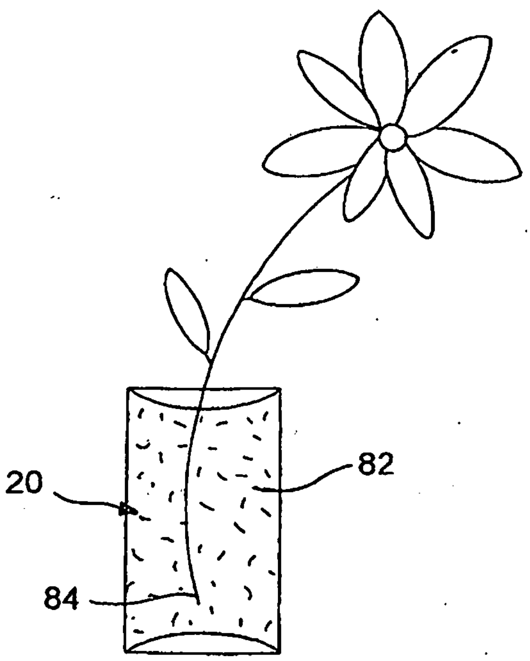


FIG. 18

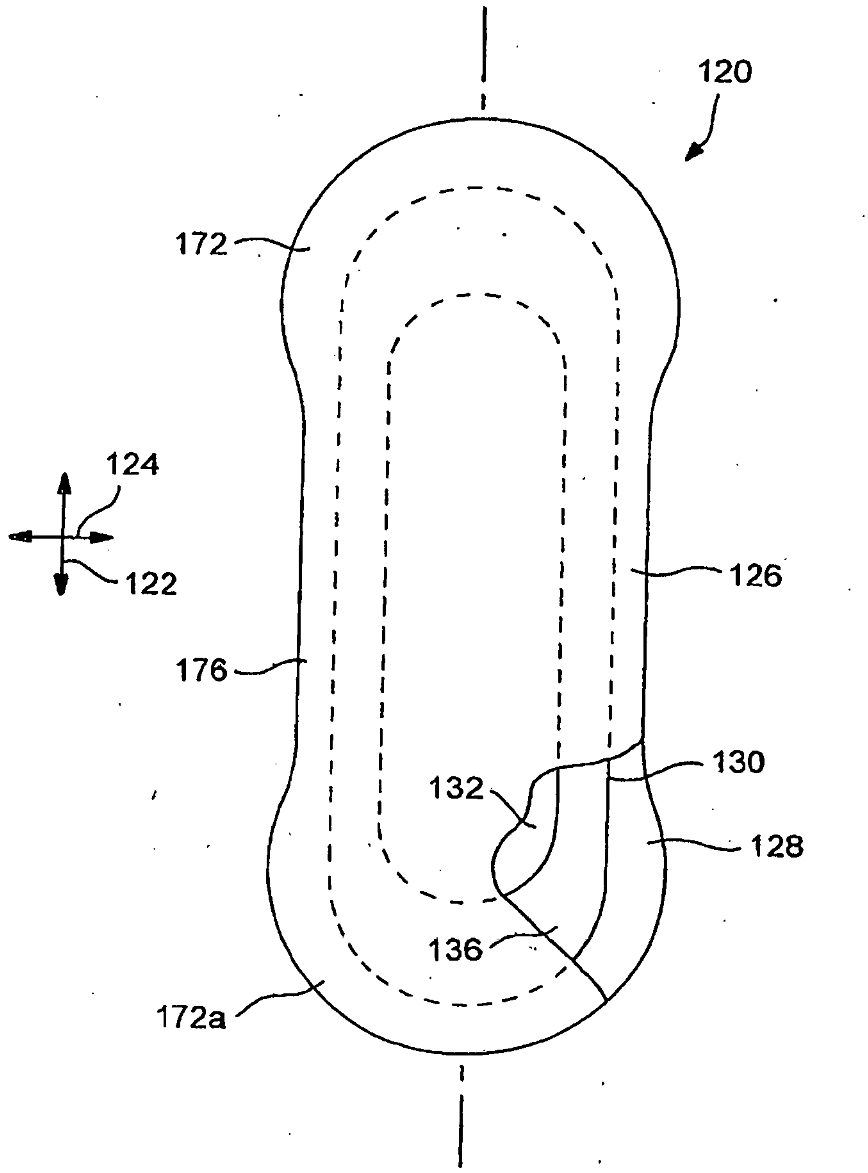


FIG. 19

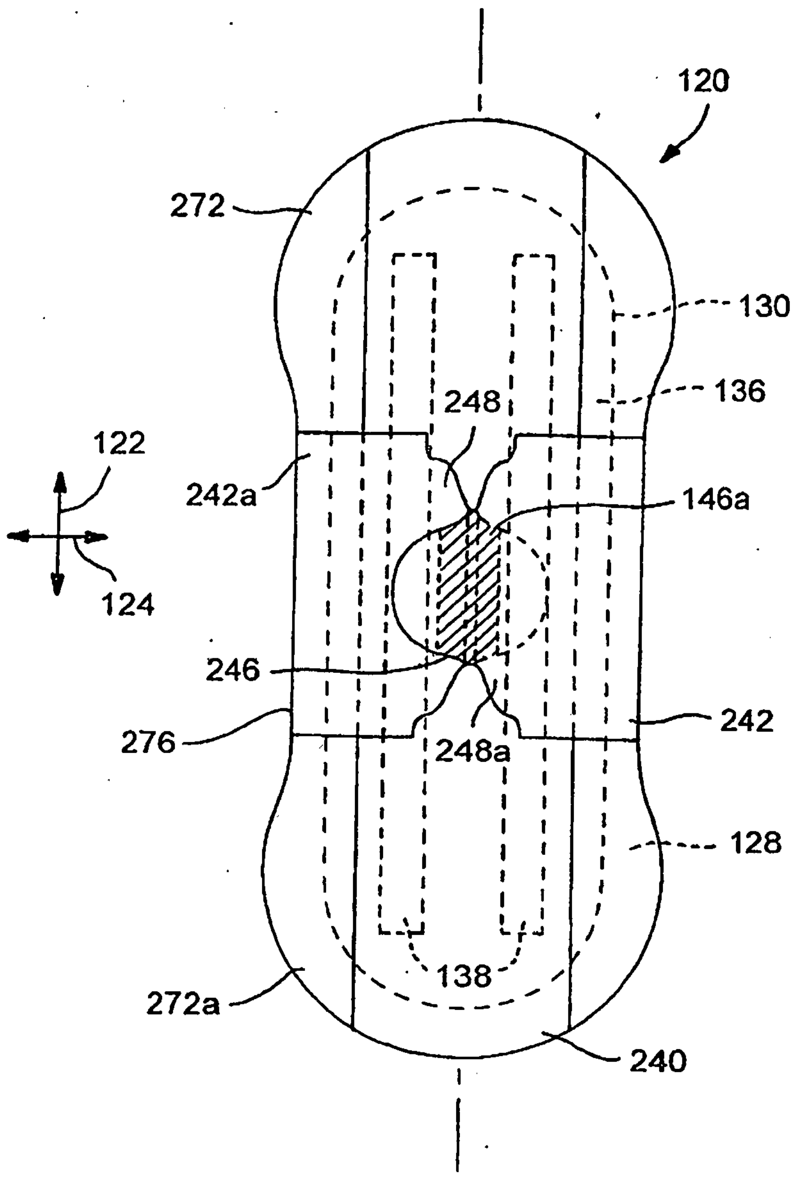
120
272
130
136
248
122
242a
146a
124
246
276
242
248a
128
138
272a
240

FIG. 19A

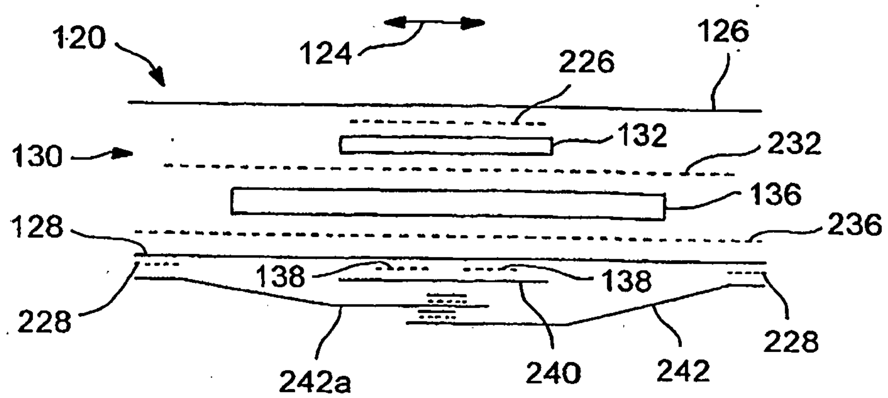


FIG. 19B

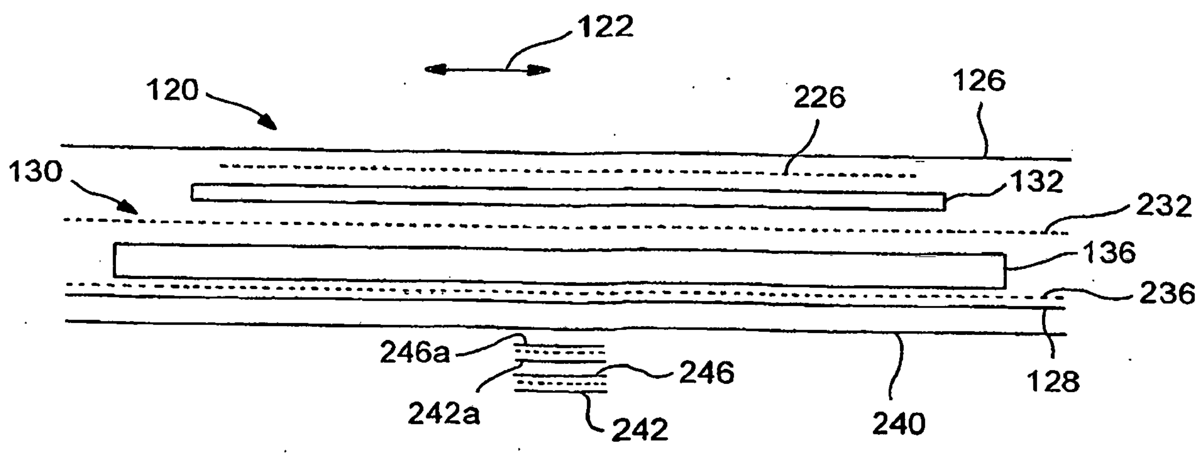


FIG. 19C

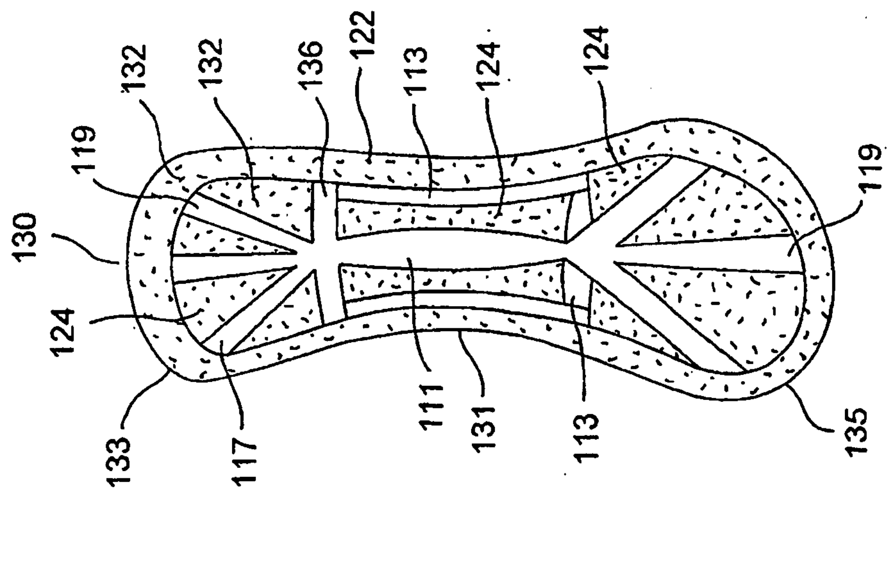


FIG. 20

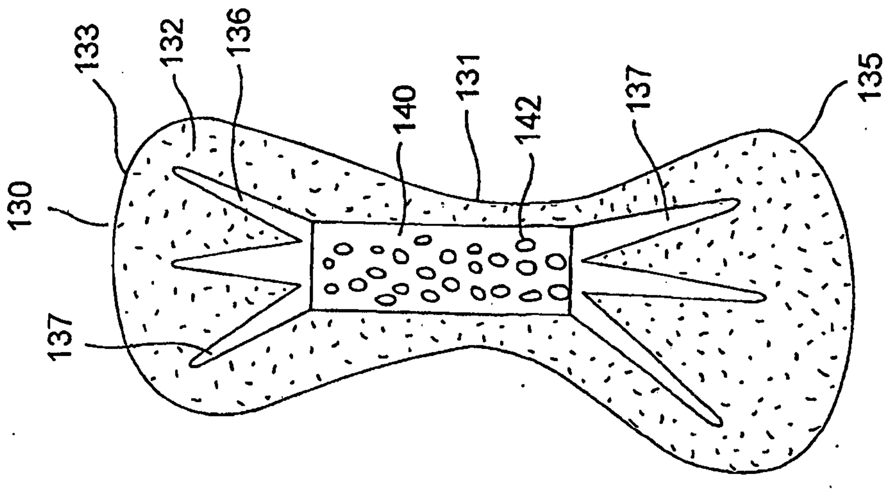


FIG. 21

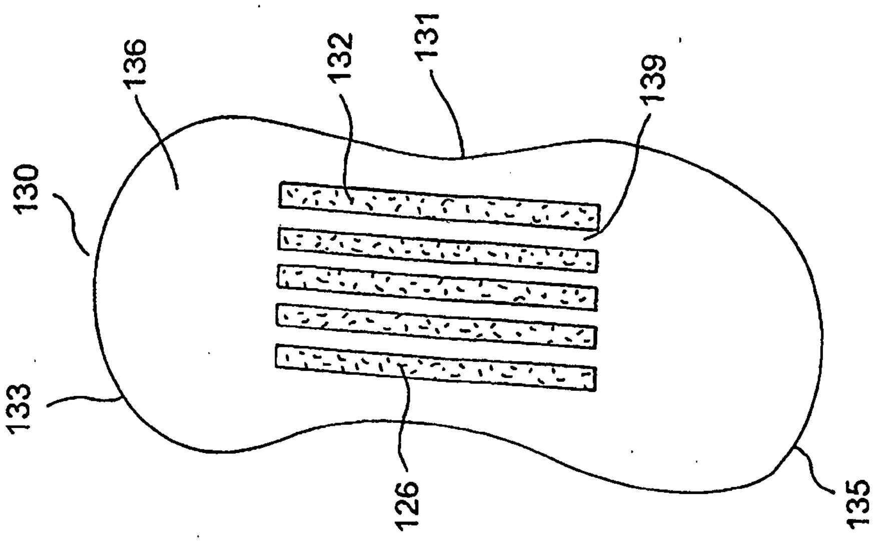


FIG. 22

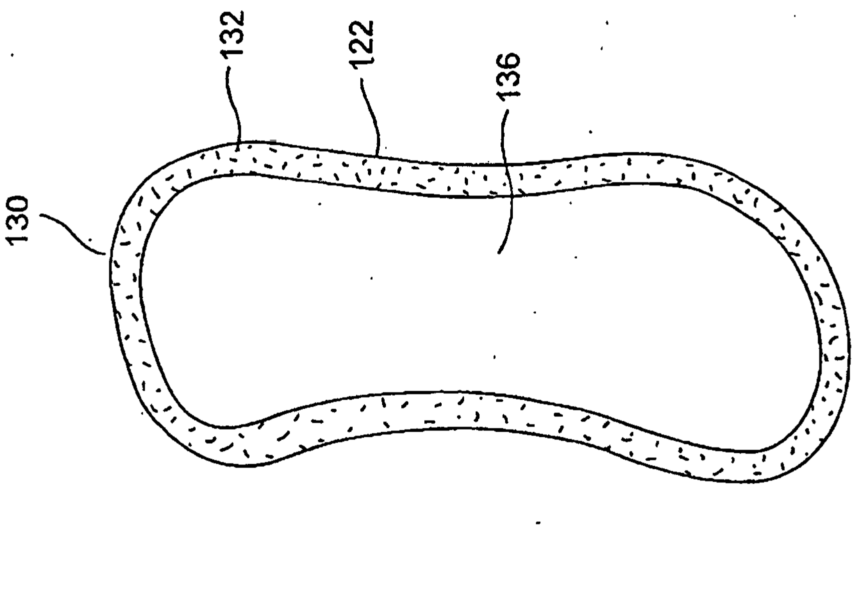


FIG. 23

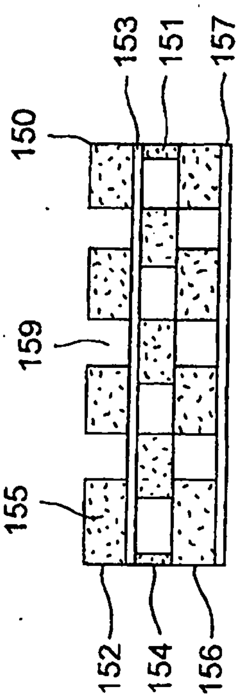


FIG. 24

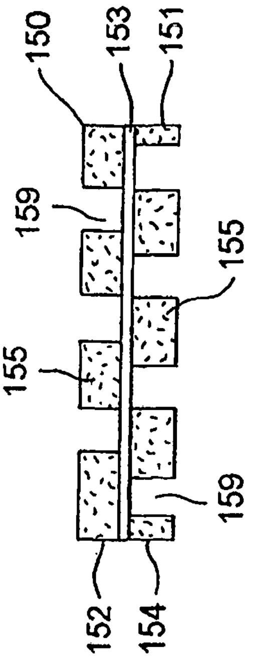


FIG. 25

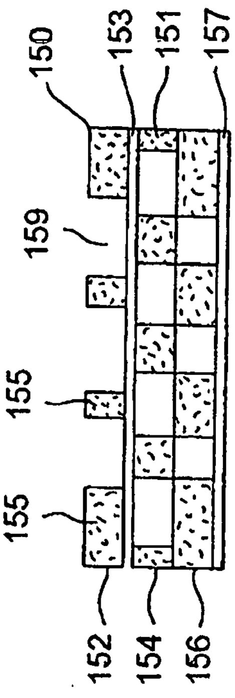


FIG. 26

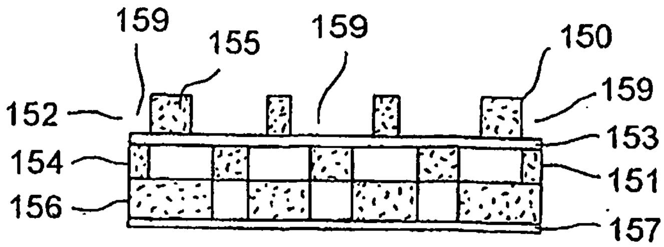


FIG. 27

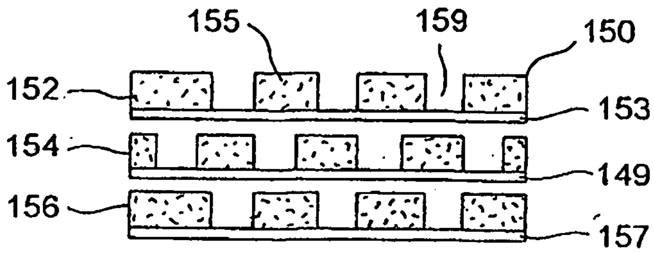


FIG. 28

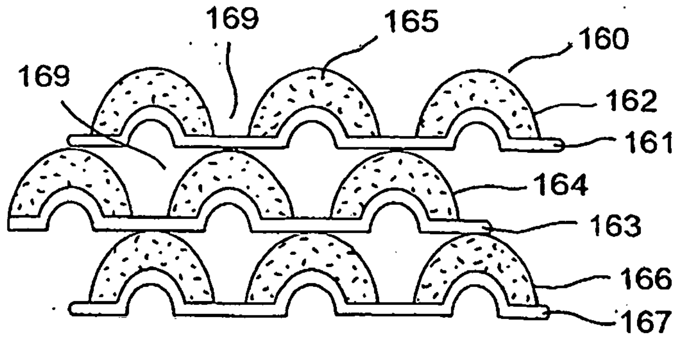


FIG. 29

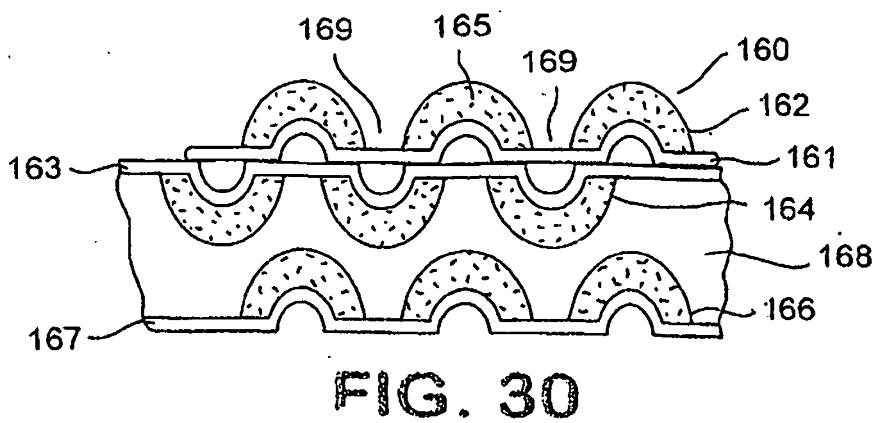


FIG. 30

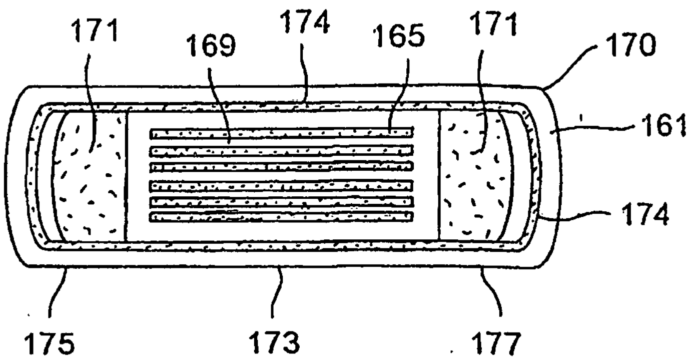


FIG. 31

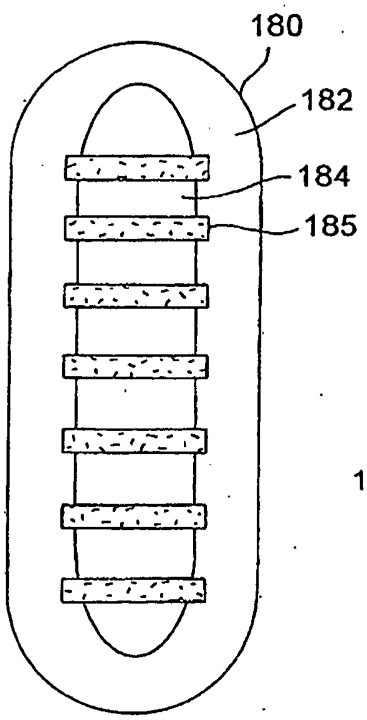


FIG. 32

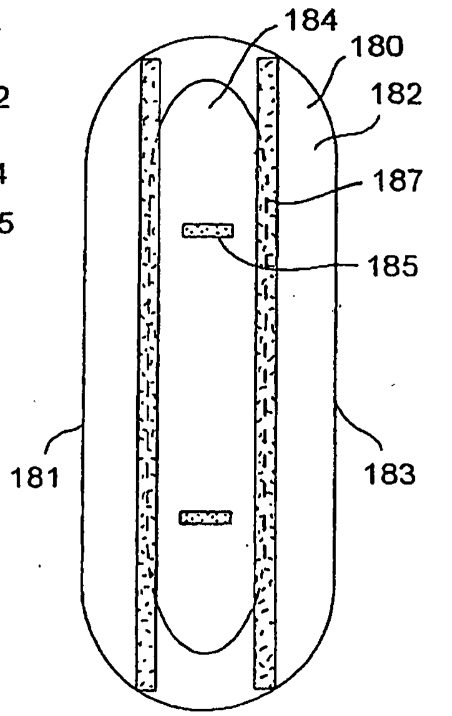


FIG. 33

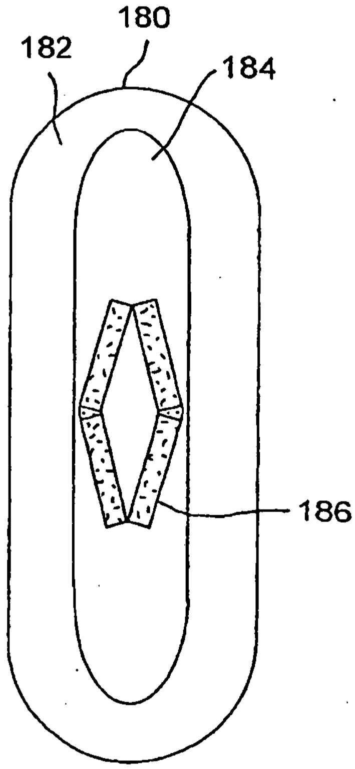


FIG. 34

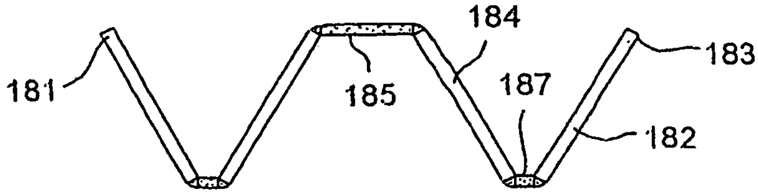


FIG. 35

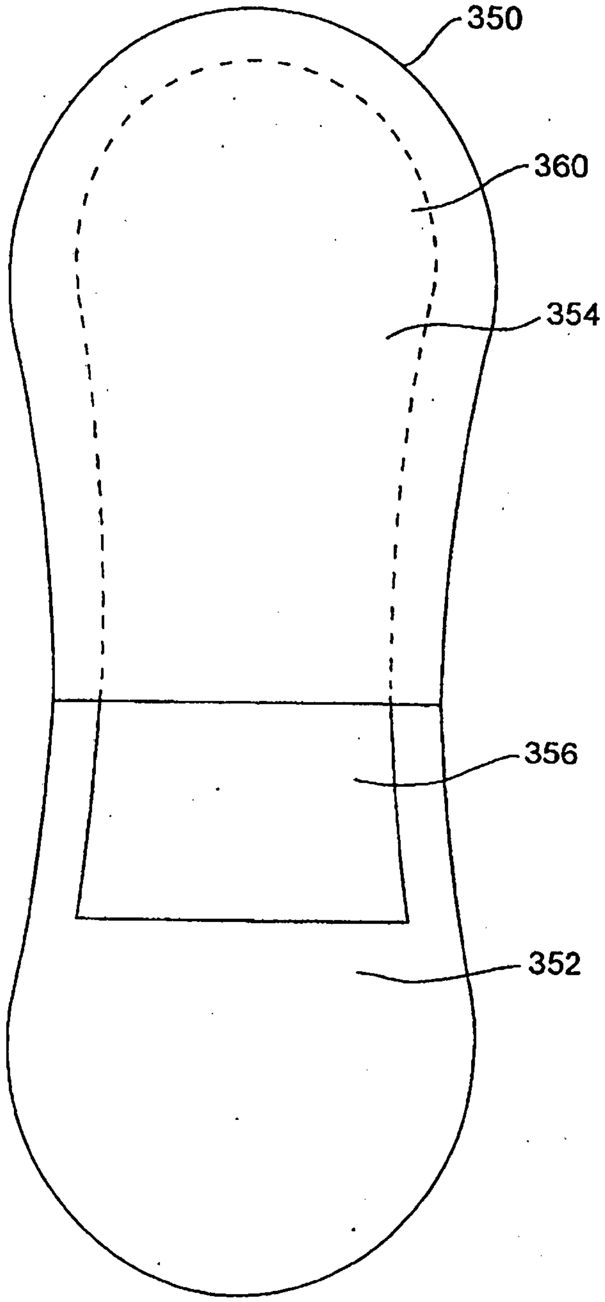


FIG. 36

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6054523 A, Braun
- US 5196470 A, Anderson
- US 5389728 A, Prejean
- US 5112919 A, Furrer
- US 4593071 A
- US 5047476 A
- US 4767820 A
- US 4753993 A
- US 4579913 A
- US 4575535 A
- US 4551504 A
- US 4526930 A
- US 4493924 A
- US 4489029 A
- US 4446279 A
- US 4440907 A
- US 4434272 A
- US 4408011 A
- US 4369289 A
- US 4353997 A
- US 4343917 A
- US 4328323 A
- US 4291136 A
- WO 02053644 A
- US 5204404 A, Werner
- US 4704116 A, Enloe
- US 4940464 A, Van Gompel
- US 37994203 A
- US 39211603 A
- US 5364382 A